(19) Europäisches Patentamt
European Patent Office
Office européen des brevets

(11) Veröffentlichungsnummer: **0 302 328**
**A2**

# (12) EUROPÄISCHE PATENTANMELDUNG

(21) Anmeldenummer: 88111846.7

(51) Int. Cl.⁴: C07D 231/40 , A01N 43/56

(22) Anmeldetag: 22.07.88

(30) Priorität: 03.08.87 DE 3725661

(43) Veröffentlichungstag der Anmeldung:
08.02.89 Patentblatt 89/06

(84) Benannte Vertragsstaaten:
BE CH DE FR GB IT LI NL

(71) Anmelder: BAYER AG
Konzernverwaltung RP Patentabteilung
D-5090 Leverkusen 1 Bayerwerk(DE)

(72) Erfinder: Gehring, Reinhold, Dr.
Dasnöckel 49
D-5600 Wuppertal 11(DE)
Erfinder: Schallner, Otto, Dr.
Noldeweg 22
D-4019 Monheim(DE)
Erfinder: Stetter, Jörg, Dr.
Gellertweg 4
D-5600 Wuppertal 1(DE)
Erfinder: Marhold, Albrecht, Dr.
Carl-Duisberg-Strasse 329
D-5090 Leverkusen 1(DE)
Erfinder: Santel, Hans-Joachim, Dr.
Grünstrasse 9a
D-5090 Leverkusen 1(DE)
Erfinder: Schmidt, Robert R., Dr.
Im Waldwinkel 110
D-5060 Bergisch Gladbach 2(DE)
Erfinder: Lürssen, Klaus, Dr.
August-Kierspel-Strasse 145
D-5060 Bergisch Gladbach 2(DE)
Erfinder: Strang, Harry, Dr.
Unterdorfstrasse 6A
D-4000 Düsseldorf 31(DE)

## (54) 1-Arylpyrazole.

(57) Die Erfindung betrifft 1-Arylpyrazole der Formel (I)

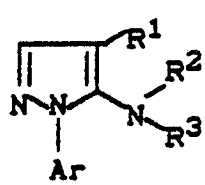

(I)

in welcher
$R^1$ für Wasserstoff oder Nitro steht,
$R^2$ für Wasserstoff, für einen Rest $-\overset{X}{\overset{\|}{C}}-R^4$ oder einen Rest $-S(O)_n-R^5$ steht,

EP 0 302 328 A2

$R^3$ für Alkyl, für einen Rest $-\overset{X}{\overset{\|}{C}}-R^4$ oder für einen Rest $-S(O)_n-R^5$ steht und außerdem für den Fall, daß $R^2$ für einen Rest $-SO_2-R^5$ steht, auch für ein salzartig gebundenes anorganisches oder organisches Kation steht,
und die übrigen Substituenten die in der Beschreibung angegebene Bedeutung haben, mehrere Verfahren zu ihrer Herstellung sowie ihre Verwendung als Herbizide und Pflanzenwuchsregulatoren.

# 1-Arylpyrazole

Die Erfindung betrifft neue 1-Arylpyrazole, mehrere Verfahren zu ihrer Herstellung und ihre Verwendung als Herbizide und Pflanzenwachstumsregulatoren.

Es ist bekannt, daß bestimmte 1-Arylpyrazole wie beispielsweise das 5-Chloracetamido-1-(2,6-dichlor-4-trifluormethylphenyl)-4-nitro-pyrazol herbizide, insbesondere auch selektiv-herbizide Eigenschaften besitzen (vgl. z.B. DE-OS 34 02 308).

Die herbizide Wirksamkeit der vorbekannten Verbindungen gegenüber Schadpflanzen ist jedoch, ebenso wie ihre Verträglichkeit gegenüber wichtigen Kulturpflanzen, nicht in allen Anwendungsbereichen völlig zufriedenstellend.

Es wurde neue 1-Arylpyrazole der allgemeinen Formel (I),

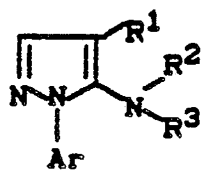

(I)

in welcher

$R^1$ für Wasserstoff oder Nitro steht,

$R^2$ für Wasserstoff, für einen Rest $-\overset{X}{\overset{\|}{C}}-R^4$ oder einen Rest $-S(O)_n-R^5$ steht,

$R^3$ für Alkyl, für einen Rest $-\overset{X}{\overset{\|}{C}}-R^4$ oder für einen Rest $-S(O)_n-R^5$ steht und außerdem für den Fall, daß $R^2$ für einen Rest $-SO_2-R^5$ steht, auch für ein salzartig gebundenes anorganisches oder organisches Kation steht,

wobei

$R^4$ für Wasserstoff, Alkyl, Alkenyl, Alkinyl, Monohalogenalkyl, Alkoxyalkyl, Halogenalkoxyalkyl, Alkylthioalkyl, Alkoxy, Alkylthio, Alkylsulfonylalkyl, Alkylsulfinylalkyl, Alkylamino, Dialkylamino für gegebenenfalls substituiertes Cycloalkyl, für gegebenenfalls substituiertes Aryl, für gegebenenfalls substituiertes Aryloxy, für gegebenenfalls substituiertes Arylthio oder für gegebenenfalls substituiertes Arylamino steht,

X für Sauerstoff oder Schwefel steht,

$R^5$ für Alkyl, Halogenalkyl oder für gegebenenfalls substituiertes Aryl steht,

n für eine Zahl 0, 1 oder 2 steht und

Ar für einen der Reste

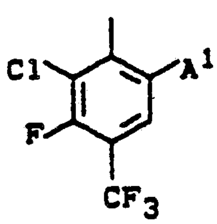 ; 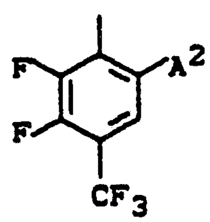 ; 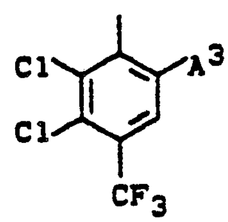 ;

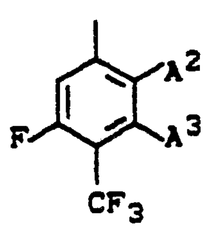 ; 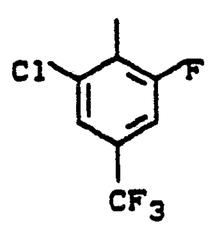 oder 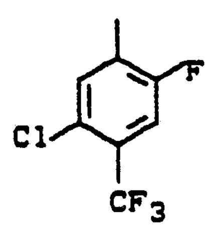

steht, wobei

$A^1$ für Wasserstoff, Fluor oder Chlor steht,

$A^2$ für Fluor oder Chlor steht und

$A^3$ für Wasserstoff oder Fluor steht,
wobei jedoch die Verbindungen 5-Ethoxycarbonylamino-1-(2,3-dichlor-4-trifluormethylphenyl)-4-nitro-pyrazol und 5-Acetamido-1-(2,5-difluor-4-trifluormethylphenyl)-pyrazol ausgenommen sind,
gefunden.

Weiterhin wurde gefunden, daß die neuen 1-Arylpyrazole der allgemeinen Formel (I),

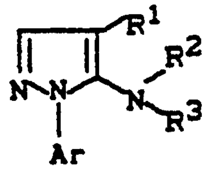

(I)

in welcher
$R^1$ für Wasserstoff oder Nitro steht,
$R^2$ für Wasserstoff, für einen Rest $-\overset{X}{\overset{\|}{C}}-R^4$ oder einen Rest $-S(O)_n-R^5$ steht,
$R^3$ für Alkyl, für einen Rest $-\overset{X}{\overset{\|}{C}}-R^4$ oder für einen Rest $-S(O)_n-R^5$ steht und außerdem für den Fall, daß $R^2$ für einen Rest $-SO_2-R^5$ steht, auch für ein salzartig gebundenes anorganisches oder organisches Kation steht,
wobei
$R^4$ für Wasserstoff, Alkyl, Alkenyl, Alkinyl, für Monohalogenalkyl Alkoxyalkyl, Halogenalkoxyalkyl, Alkylthioalkyl, Alkylsulfonyl alkyl, Alkylsulfinylalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,
X für Sauerstoff oder Schwefel steht,
$R^5$ für Alkyl, Halogenalkyl oder für gegebenenfalls substituiertes Aryl steht,
n für eine Zahl 0, 1 oder 2 steht und
Ar für einen der Reste

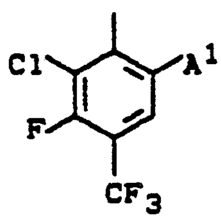 ; 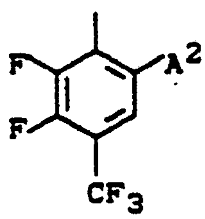 ; 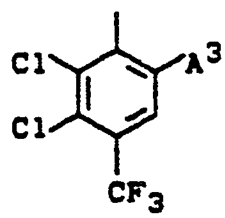 ;

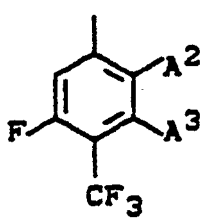 ; 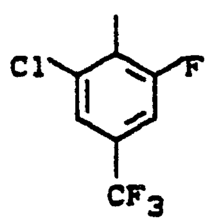 oder 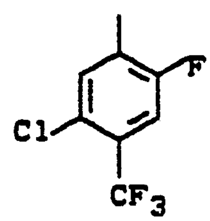

steht, wobei
$A^1$ für Wasserstoff, Fluor oder Chlor steht,
$A^2$ für Fluor oder Chlor steht und
$A^3$ für Wasserstoff oder Fluor steht,
wobei jedoch die Verbindungen 5-Ethoxycarbonylamino-1-(2,3-dichlor-4-trifluormethylphenyl)-4-nitro-pyrazol und 5-Acetamido-1-(2,5-difluor-4-trifluormethylphenyl)-pyrazol ausgenommen sind,
nach folgenden Verfahren herstellbar sind:

(a) Man erhält 1-Arylpyrazole der Formel (Ia),

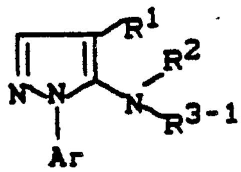

(Ia)

in welcher

$R^{3-1}$ für Alkyl, für einen Rest $-\overset{X}{\overset{\|}{C}}-R^4$ oder für einen Rest $-S(O)_n-R^5$ steht und $R^1$, $R^2$, $R^4$, $R^5$, X, n und Ar die oben angegebene Bedeutung haben, wenn man 1-Arylpyrazole der Formel (II),

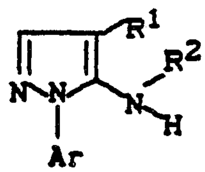

(II)

in welcher

$R^1$, $R^2$ und Ar die oben angegebene Bedeutung haben,

(a-α) mit Acylierungsmitteln der Formel (III),

$$R^4-\underset{\underset{X}{\|}}{C}-E^1$$

(III)

in welcher

$E^1$ für eine elektronenanziehende Abgangsgruppe steht und $R^4$ und X die oben angegebene Bedeutung haben, oder

(a-β) mit Sulfenylierungs-, Sulfinylierungs- oder Sulfonylierungsmitteln der Formel (IV),

$R^5-S(O)_n-E^2$ (IV)

in welcher

$E^2$ für eine elektronenanziehende Abgangsgruppe steht und $R^5$ und n die oben angegebene Bedeutung haben, oder

(a-γ) mit Alkylierungsmitteln der Formel (V),

$R^{3-2}-E^3$ (V)

in welcher

$R^{3-2}$ für Alkyl steht und

$E^3$ für eine elektronenanziehende Abgangsgruppe steht,

jeweils gegebenenfalls in Gegenwart eines Verdünnungsmittels sowie gegebenenfalls in Gegenwart eines Reaktionshilfsmittels umsetzt;

(b) man erhält 1-Arylpyrazole der Formel (Ib),

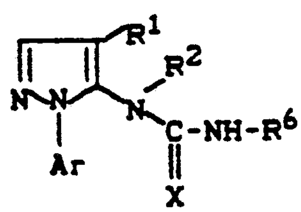

(Ib)

in welcher

$R^6$ für Alkyl oder für gegebenenfalls substituiertes Aryl steht und
$R^1$, $R^2$, X und Ar die oben angegebene Bedeutung haben,
wenn man 1-Arylpyrazole der Formel (II),

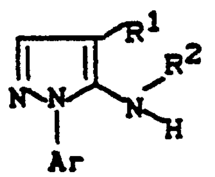

(II)

in welcher
$R^1$, $R^2$ und Ar die oben angegebene Bedeutung haben,
mit Iso(thio)cyanaten der Formel (VI),

$R^6$-N=C=X (VI)

in welcher
$R^6$ und X die oben angegebene Bedeutung haben,
gegebenenfalls in Gegenwart eines Verdünnungsmittels und gegebenenfalls in Gegenwart eines Reaktionshilfsmittels umsetzt;

(c) man erhält 1-Arylpyrazole der Formel (Ic),

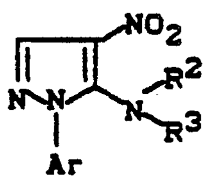

(Ic)

in welcher
$R^2$, $R^3$ und Ar die oben angegebene Bedeutung haben,
wenn man 1-Arylpyrazole der Formel (Iz),

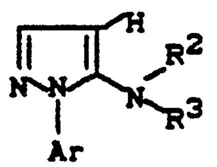

(Iz)

in welcher
$R^2$, $R^3$ und Ar die oben angegebene Bedeutung haben,
mit Salpetersäure gegebenenfalls in Gegenwart eines Verdünnungsmittels und gegebenenfalls in Gegenwart eines Reaktionshilfsmittels umsetzt;

(d) man erhält 1-Arylpyrazole der Formel (Id),

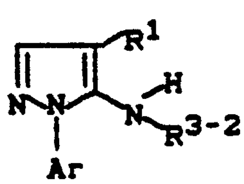

(Id)

in welcher
$R^{3-2}$ für Alkyl steht und
$R^1$ und Ar die oben angegebene Bedeutung haben,
wenn man 1-Arylpyrazole der Formel (Iy),

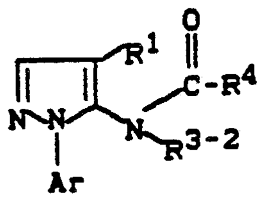

(Iy)

in welcher
$R^1$, $R^{3-2}$, $R^4$ und Ar die oben angegebene Bedeutung haben,
gegebenenfalls in Gegenwart eines Verdünnungsmittels und gegebenenfalls in Gegenwart eines Reaktionshilfsmittels an der Aminogruppe in 5-Position des Pyrazolringes hydrolysiert;

(e) man erhält 1-Arylpyrazole der Formel (Ie),

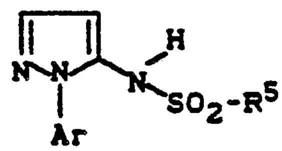

(Ie)

in welcher
$R^5$ und Ar die oben angegebene Bedeutung haben,
wenn man 1-Arylpyrazole der Formel (Ix),

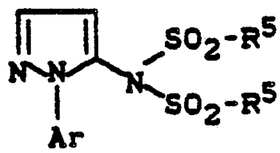

(Ix)

in welcher
$R^5$ und Ar die oben angegebene Bedeutung haben,
gegebenenfalls in Gegenwart eines Verdünnungsmittels und gegebenenfalls in Gegenwart eines Reaktionshilfsmittels an der Aminogruppe in 5-Position des Pyrazolringes hydrolysiert;

(f) man erhält 1-Arylpyrazole der Formel (If),

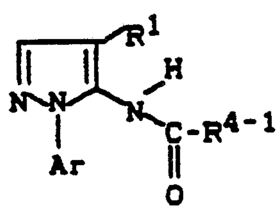

(If)

in welcher
$R^{4-1}$ für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht und
$R^1$ und Ar die oben angegebene Bedeutung haben,
wenn man 1-Arylpyrazole der Formel (Iw),

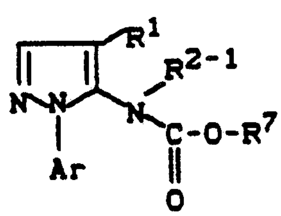

(Iw)

in welcher
$R^{2-1}$ für Wasserstoff oder für einen Rest $-\underset{\underset{O}{\|}}{C}-O-R^7$
steht,
$R^7$ für gegebenenfalls substituiertes Aryl steht und
$R^1$ and Ar die oben angegebene Bedeutung haben,
mit Alkoholen, Aminen oder Thiolen der Formel (VII),

$R^{4-1}$-H (VII)

in welcher
$R^{4-1}$ die oben angegebene Bedeutung hat,
gegebenenfalls in Gegenwart eines Verdünnungsmittels und gegebenenfalls in Gegenwart eines Reaktionshilfsmittels umsetzt;

(g) man erhält 1-Arylpyrazole der Formel (Ig),

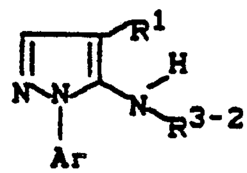

(Ig)

in welcher
$R^{3-2}$ für Alkyl steht und
$R^1$ und Ar die oben angegebene Bedeutung haben,
wenn man 5-Halogen-1-aryl-pyrazole der Formel (VIII),

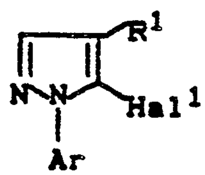

(VIII)

in welcher
$Hal^1$ für Halogen steht und
$R^1$ und Ar die oben angegebene Bedeutung haben,
mit Aminen der Formel (IX),

$H_2N - R^{3-2}$ (IX)

in welcher
$R^{3-2}$ die oben angegebene Bedeutung hat,
gegebenenfalls in Gegenwart eines Verdünnungsmittels und gegebenenfalls in Gegenwart eines Reaktionshilfsmittels umsetzt;

(h) man erhält 1-Arylpyrazole der Formel (Ih),

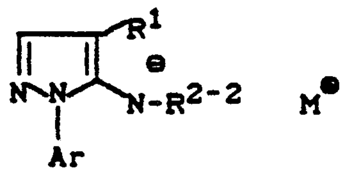

(Ih)

in welcher
$R^{2-2}$ für einen Rest $-SO_2-R^5$ steht,
$M^{\oplus}$ für ein Äquivalent eines anorganischen oder organischen Kations steht und

$R^1$, $R^5$ und Ar die oben angegebene Bedeutung haben,
wenn man 1-Arylpyrazole der Formel (Iv),

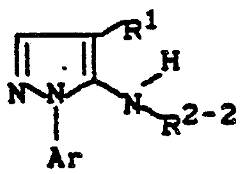

(Iv)

in welcher
$R^1$, $R^{2-2}$ und Ar die oben angegebene Bedeutung haben,
mit Salzen der Formel (X),

$M^{\oplus}$-$G^{\ominus}$ (X)

in welcher
$M^{\oplus}$ die oben angegebene Bedeutung hat und
$G^{\ominus}$ für ein Äquivalent eines geeigneten Gegenions steht,
oder mit primären, sekundären oder tertiären Aminen, gegebenenfalls in Gegenwart eines Verdünnungsmittels umsetzt.

Schließlich wurde gefunden, daß die neuen 1-Arylpyrazole der allgemeinen Formel (I) herbizide, insbesondere auch selektiv-herbizide und pflanzenwachstumsregulierende Eigenschaften besitzen.

Überraschenderweise zeigen die erfindungsgemäßen 1-Arylpyrazole der allgemeinen Formel (I) eine deutlich bessere herbizide Wirksamkeit, bei vergleichbar guter Nutz pflanzenselektivität, als die aus dem Stand der Technik bekannten 1-Arylpyrazole, wie beispielsweise das 5-Chloracetamido-1-(2,6-dichlor-4-trifluormethylphenyl)-4-nitro-pyrazol, welche chemisch und wirkungsmäßig naheliegende Verbindungen sind.

Die erfindungsgemäßen 1-Arylpyrazole sind durch die Formel (I) allgemeinen definiert. Bevorzugt sind Verbindungen der Formel (I), bei welchen
$R^1$ für Wasserstoff oder Nitro steht,
$R^2$ für Wasserstoff, für einen Rest $-\overset{X}{\overset{\|}{C}}-R^4$ oder für einen Rest $-S(O)_n-R^5$ steht,
$R^3$ für geradkettiges oder verzweigtes Alkyl mit 1 bis 6 Kohlenstoffatomen, für einen Rest $-\overset{X}{\overset{\|}{C}}-R^4$ oder für einen Rest $-S(O)_n-R^5$ steht und außerdem für den Fall, daß $R^2$ für einen Rest $-SO_2-R^5$ steht, auch für ein salzartig gebundenes Äquivalent eines Alkali-oder Erdalkali-oder Übergangsmetallkations oder für ein gegebenenfalls durch $C_1$-$C_6$-Alkyl oder Phenyl substituiertes Ammoniumion steht, wobei
$R^4$ für Wasserstoff, für geradkettiges oder verzweigtes Alkyl mit 1 bis 12 Kohlenstoffatomen, für jeweils geradkettiges oder verzweigtes Alkenyl oder Alkinyl mit jeweils 2 bis 6 Kohlenstoffatomen, für jeweils geradkettiges oder verzweigtes Monohalogenalkyl, Alkoxyalkyl, Halogenalkoxyalkyl ,Alkylthioalkyl, Alkoxy, Alkylthio, Alkylsulfonylalkyl, Alkylsulfinylalkyl, Alkylamino oder Dialkylamino mit jeweils 1 bis 4 Kohlenstoffatomen in den einzelnen Alkylteilen und im Fall des Halogenalkoxyalkyl, mit 1 bis 9 gleichen oder verschiedenen Halogenatomen steht, außerdem für gegebenenfalls einfach oder mehrfach, gleich oder verschieden durch Halogen und/oder jeweils geradkettiges oder verzweigtes Alkyl oder Halogenalkyl mit jeweils 1 bis 4 Kohlenstoffatomen und im Fall des Halogenalkyl mit 1 bis 9 gleichen oder verschiedenen Halogenatomen substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten jeweils in Frage kommen: Halogen, jeweils geradkettiges oder verzweigtes Alkyl oder Alkoxy mit jeweils 1 bis 4 Kohlenstoffatomen sowie geradkettiges oder verzweigtes Halogenalkyl mit 1 bis 4 Kohlenstoffatomen und 1 bis 9 gleichen oder verschiedenen Halogenatomen.
X für Sauerstoff oder Schwefel steht.
$R^5$ für jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder für gegebenenfalls einfach oder mehrfach, gleich oder verschieden substituiertes Phenyl steht, wobei als Phenylsubstituenten in Frage kommen: Halogen, jeweils geradkettiges oder verzweigtes Alkyl oder Alkoxy mit jeweils 1 bis 4 Kohlenstoffatomen sowie geradkettiges oder verzweigtes Halogenalkyl mit 1 bis 4 Kohlenstoffatomen und 1 bis 9 gleichen oder verschiedenen Halogenatomen,
n für eine Zahl 0, 1 oder 2 steht und

Ar für einen der Reste

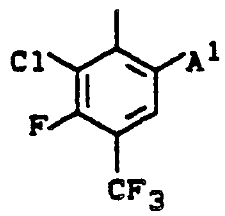 ; 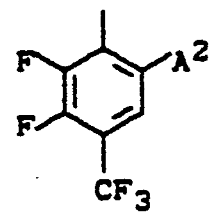 ; 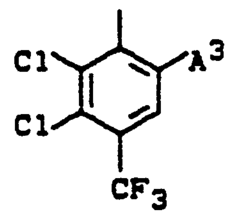 ;

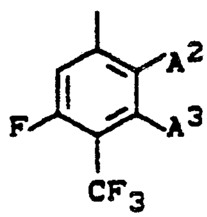 ; 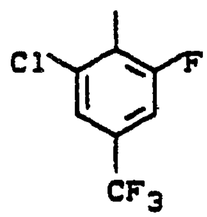 oder 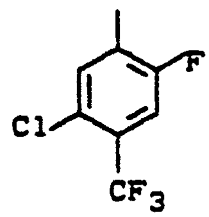

steht, wobei
$A^1$ für Wasserstoff, Fluor oder Chlor steht,
$A^2$ für Fluor oder Chlor steht und
$A^3$ für Wasserstoff oder Fluor steht
wobei jedoch die Verbindungen 5-Ethoxycarbonylamino-1-(2,3-dichlor-4-trifluormethylphenyl)-5-nitro-pyrazol und 5-Acetamido-1-(2,5-difluor-4-trifluormethylphenyl)-pyrazol ausgenommen sind.

Besonders bevorzugt sind Verbindungen der Formel (I), bei welchen
$R^1$ für Wasserstoff oder Nitro steht,
$R^2$ für Wasserstoff, für einen Rest $-\underset{\underset{X}{\|}}{C}-R^4$,
oder für einen Rest $-S(O)_n-R^5$ steht,
$R^3$ für Methyl, Ethyl, n- oder i-Propyl, n-, i-, s-oder t-Butyl, für einen Rest $-\underset{\underset{X}{\|}}{C}-R^4$
oder für einen Rest $-S(O)_n-R^5$ steht, und außerdem für den Fall, daß $R^2$ für einen Rest $-SO_2-R^5$ steht, auch für ein salzartig gebundenes Äquivalent eines Natrium-, Kalium-, Magnesium-, Calcium-, Barium-, Kupfer-, Zink-, Mangan-, Zinn-, Eisen-, Cobalt- oder Nickel-ions steht, oder für ein gegebenenfalls ein- bis dreifach, gleich oder verschieden durch Methyl, Ethyl, n- oder i-Propyl, n-, i-, s-oder t-Butyl oder Phenyl substituiertes Ammoniumion steht, wobei
$R^4$ für Wasserstoff, Methyl, Ethyl, n- oder i-Propyl, n-, i-, s- oder t-Butyl, für jeweils geradkettiges oder verzweigtes Pentyl, Hexyl, Heptyl, Octyl, Nonyl, Decyl, Undecyl, für Vinyl, Allyl, Propargyl, n- oder i-Butenyl, Methoxymethyl, Ethoxymethyl, Methoxyethyl, Ethoxyethyl, n-Propoxymethyl, Isopropoxymethyl, Methylthiomethyl, Methoxy, Ethoxy, Methylthio, Ethylthio, Methylsulfonylmethyl, Methylsulfonylethyl, Ethylsulfonylmethyl, Ethylsulfonylethyl, Methylsulfinylmethyl, Methylsulfinylethyl, Ethylsulfinylmethyl, Ethylsulfinylethyl, Methylamino, Ethylamino, Dimethylamino, Diethylamino, Chlormethyl, Iodmethyl, Brommethyl, 1-Chlorethyl, 2-Chlorethyl, 2-Bromethyl, 3-Chlorpropyl, für Trifluorethoxymethyl, für jeweils gegebenenfalls ein-bis vierfach, gleich oder verschieden durch Fluor, Chlor, Brom, Methyl oder Trifluormethyl substituiertes Cyclopropyl, Cyclopentyl oder Cyclohexyl oder für jeweils gegebenenfalls ein- bis dreifach, gleich oder verschieden durch Methyl, Methoxy, Chlor oder Trifluormethyl substituiertes Phenyl, Phenoxy, Phenylthio oder Phenylamino steht,
X für Sauerstoff oder Schwefel steht,
$R^5$ für Methyl, Ethyl, n- oder i-Propyl, n-, i-, s-oder t-Butyl, Chlormethyl, Dichlormethyl, Trichlormethyl, Trifluormethyl oder für gegebenenfalls ein-bis dreifach, gleich oder verschieden durch Methyl, Methoxy, Chlor oder Trifluormethyl substituiertes Phenyl steht,
n für eine Zahl 0, 1 oder 2 steht und
Ar für einen der Reste

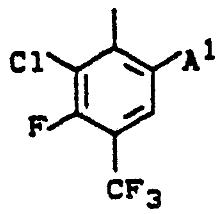 ; 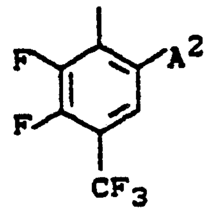 ; 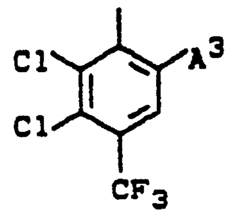 ;

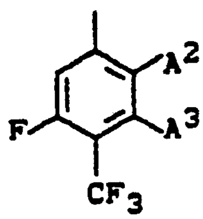 ; 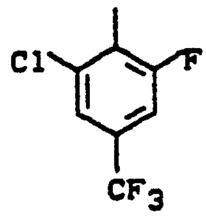 oder 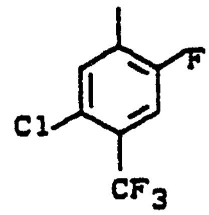

steht, wobei
$A^1$ für Wasserstoff, Fluor oder Chlor steht,
$A^2$ für Fluor oder Chlor steht und
$A^3$ für Wasserstoff oder Fluor steht,
wobei jedoch die Verbindungen 5-Ethoxycarbonylamino-1-(2,3-dichlor-4-trifluormethylphenyl)-4-nitro-pyrazol und 5-Acetamido-1-(2,5-difluor-4-trifluormethylphenyl)-pyrazol ausgenommen sind.

Ganz besonders bevorzugt sind Verbindungen der Formel (I), in welchen
$R^1$ für Nitro steht,
$R^2$ für Wasserstoff steht,
$R^3$ für Methyl, Ethyl, n- oder i-Propyl, n-, i-, s-oder t-Butyl, für einen Rest $-\underset{\underset{O}{\|}}{C}-R^4$
oder für einen Rest $-SO_2-R^5$ steht, wobei
$R^4$ für Wasserstoff, Methyl, Ethyl, n- oder i-Propyl, n-Butyl, Allyl, Propargyl, n- oder i- Butenyl, für Methoxymethyl, Ethoxymethyl, n-Propoxymethyl, Isopropoxymethyl, Methoxyethyl, Ethoxyethyl, Methylthiomethyl, Methoxy, Ethoxy, Methylthio, Ethylthio, Methylamino, Ethylamino, Dimethylamino, Diethylamino, Chlormethyl, Iodmethyl, Brommethyl, 1-Chlorethyl, 2-Chlorethyl, 2-Bromethyl, 1-Chlorpropyl, 3-Chlorpropyl, für Trifluorethoxymethyl, für Cyclopropyl, Cyclopentyl oder Cyclohexyl oder für jeweils gegebenenfalls ein- bis dreifach, gleich oder verschieden durch Methyl, Methoxy, Chlor oder Trifluormethyl substituiertes Phenyl, Phenoxy, Phenylthio oder Phenylamino steht,
$R^5$ für Methyl, Ethyl, n- oder i-Propyl, n-, i-, s-oder t-Butyl, Chlormethyl, Dichlormethyl, Trichlormethyl, Trifluormethyl oder für gegebenenfalls ein- bis dreifach, gleich oder verschieden durch Methyl, Methoxy, Chlor oder Trifluormethyl substituiertes Phenyl steht und
Ar für einen der Reste

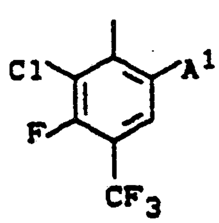 ; 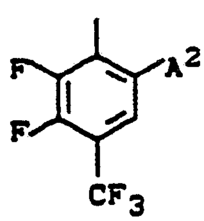 ; 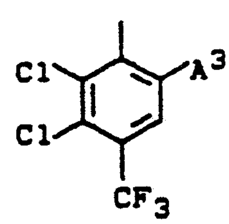 ;

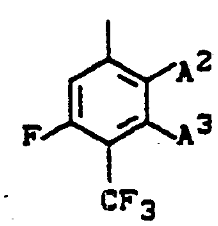 ; 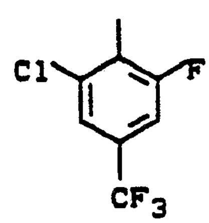 oder 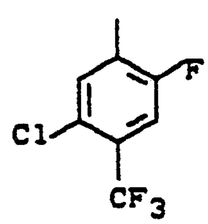

steht, wobei

A[1] für Wasserstoff, Fluor oder Chlor steht,
A[2] für Fluor oder Chlor steht und
A[3] für Wasserstoff oder Fluor steht,
wobei jedoch die Verbindungen 5-Ethoxycarbonylamino-1-(2,3-dichlor-4-trifluormethylphenyl)-4-nitro-pyrazol und 5-Acetamido-1-(2,5-difluor-4-trifluormethylphenyl)-pyrazol ausgenommen sind.

Im einzelnen seien außer den bei den Herstellungsbeispielen genannten Verbindungen die folgenden 1-Arylpyrazole der allgemeinen Formel (I) genannt:

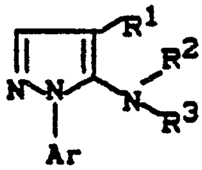

(I)

| $R^1$ | $R^2$ | $R^3$ | Ar |
|---|---|---|---|
| $NO_2$ | H | 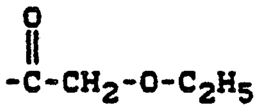 | 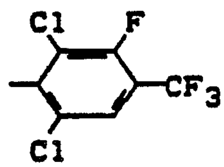 |
| $NO_2$ | H | 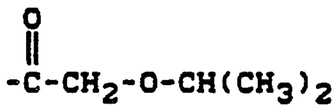 | 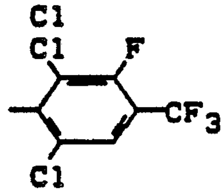 |
| $NO_2$ | H | 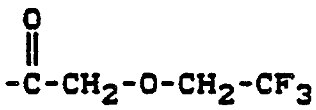 | 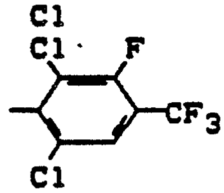 |
| $NO_2$ | H | 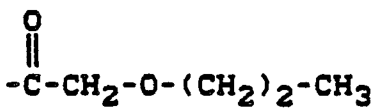 | 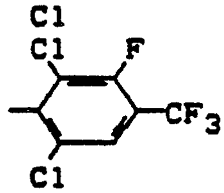 |
| $NO_2$ | H | 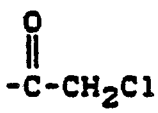 | 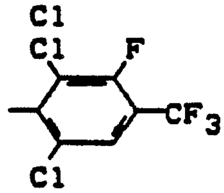 |
| $NO_2$ | H | 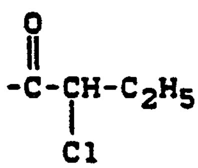 | 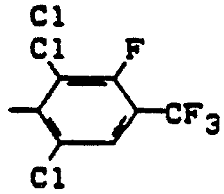 |

| $R^1$ | $R^2$ | $R^3$ | Ar |
|---|---|---|---|
| $NO_2$ | H | 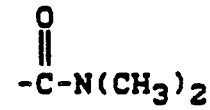 | 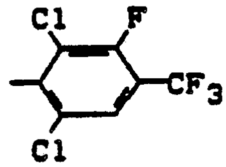 |
| $NO_2$ | H | 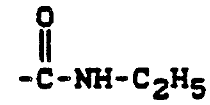 | 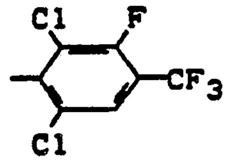 |
| $NO_2$ | H | $-SO_2-(CH_2)_3-CH_3$ | 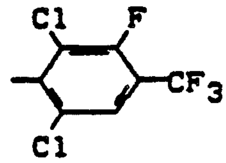 |
| $NO_2$ | H | 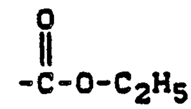 | 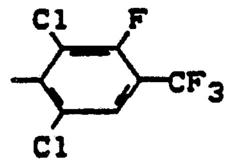 |
| $NO_2$ | H | 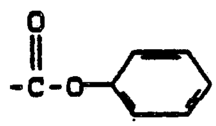 | 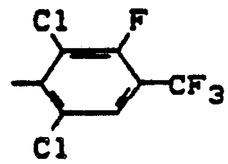 |
| $NO_2$ | H | 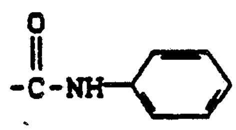 | 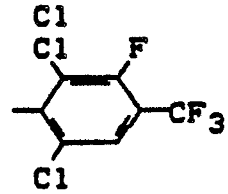 |
| $NO_2$ | H | 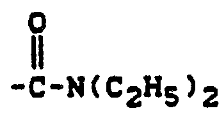 | 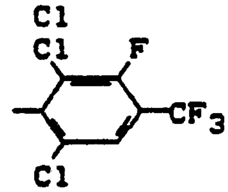 |
| $NO_2$ | H | 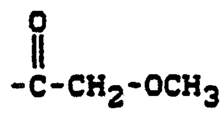 | 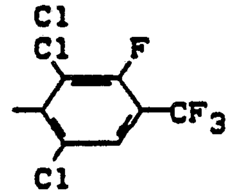 |

| $R^1$ | $R^2$ | $R^3$ | Ar |
|---|---|---|---|
| $NO_2$ | H | 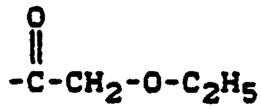 | 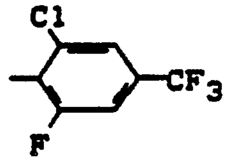 |
| $NO_2$ | H | 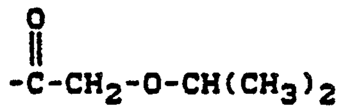 | 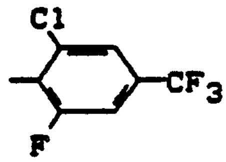 |
| $NO_2$ | H | 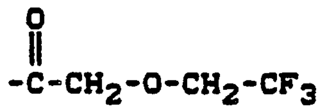 | 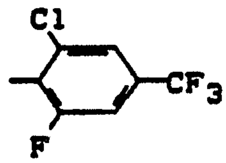 |
| $NO_2$ | H | 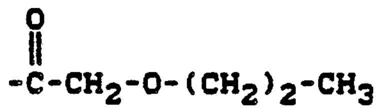 | 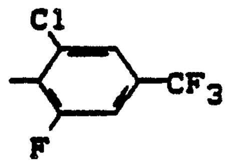 |
| $NO_2$ | H | 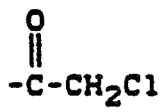 | 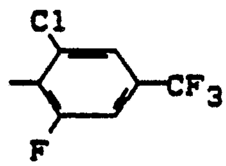 |
| $NO_2$ | H | 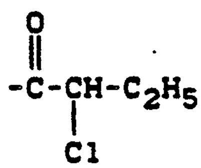 | 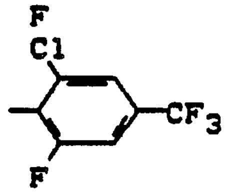 |
| $NO_2$ | H | 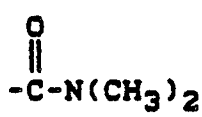 | 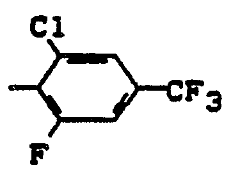 |
| $NO_2$ | H | 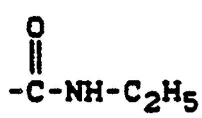 | 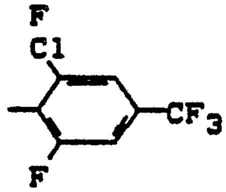 |

| $R^1$ | $R^2$ | $R^3$ | Ar |
|---|---|---|---|
| $NO_2$ | H | $-SO_2-(CH_2)_3-CH_3$ | 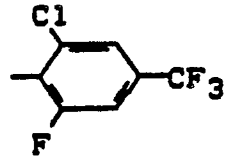 |
| $NO_2$ | H | 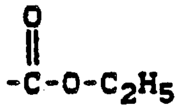 | 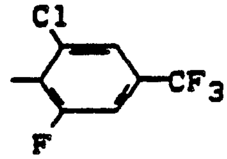 |
| $NO_2$ | H | 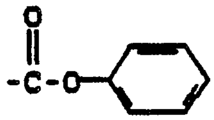 | 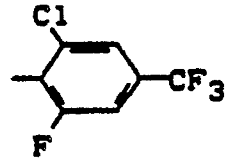 |
| $NO_2$ | H | 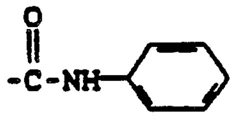 | 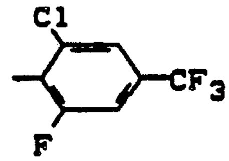 |
| $NO_2$ | H | 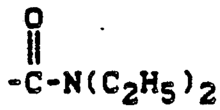 | 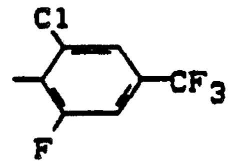 |
| $NO_2$ | H | 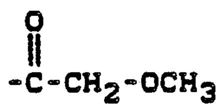 | 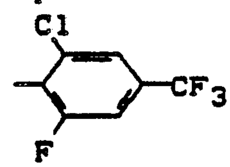 |
| $NO_2$ | H | 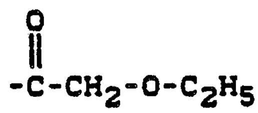 | 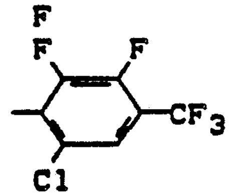 |
| $NO_2$ | H | 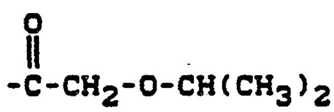 | 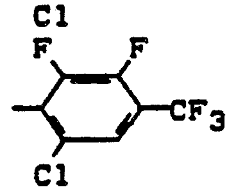 |
| $NO_2$ | H | 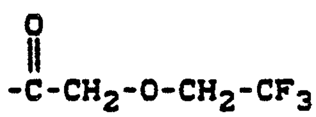 | 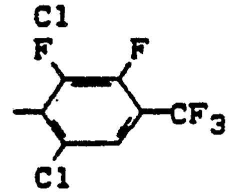 |

| $R^1$ | $R^2$ | $R^3$ | Ar |
|---|---|---|---|
| $NO_2$ | H | $-\overset{O}{\overset{\|}{C}}-CH_2-O-(CH_2)_2-CH_3$ | 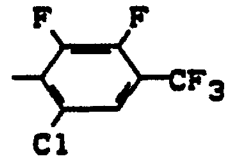 |
| $NO_2$ | H | $-\overset{O}{\overset{\|}{C}}-CH_2Cl$ | 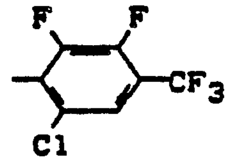 |
| $NO_2$ | H | $-\overset{O}{\overset{\|}{C}}-\underset{Cl}{\underset{\|}{C}}H-C_2H_5$ | 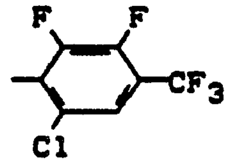 |
| $NO_2$ | H | $-\overset{O}{\overset{\|}{C}}-\underset{Cl}{\underset{\|}{C}}H-CH_3$ | 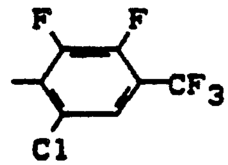 |
| $NO_2$ | H | $-\overset{O}{\overset{\|}{C}}-N(CH_3)_2$ | 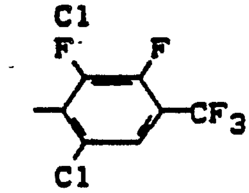 |
| $NO_2$ | H | $-\overset{O}{\overset{\|}{C}}-OCH_3$ | 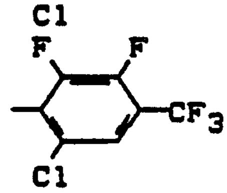 |
| $NO_2$ | H | $-\overset{O}{\overset{\|}{C}}-NH-C_2H_5$ | 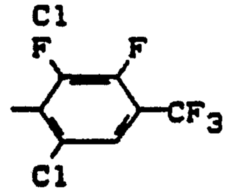 |
| $NO_2$ | H | $-\overset{O}{\overset{\|}{C}}-O-C_2H_5$ | 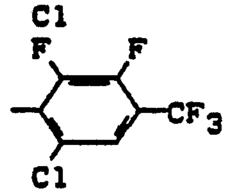 |

| $R^1$ | $R^2$ | $R^3$ | Ar |
|---|---|---|---|
| $NO_2$ | H | $-SO_2-CH_3$ | 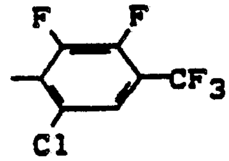 |
| $NO_2$ | H | 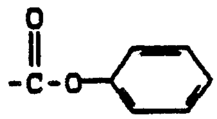 | 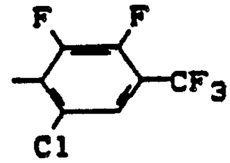 |
| $NO_2$ | H | 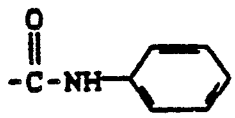 | 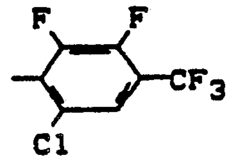 |
| $NO_2$ | H | 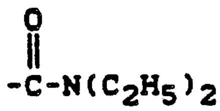 | 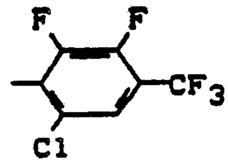 |
| $NO_2$ | H | 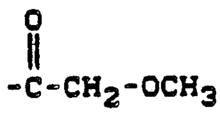 | 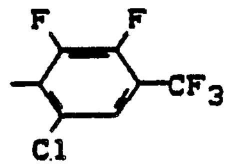 |
| $NO_2$ | H | 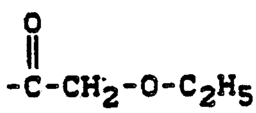 | 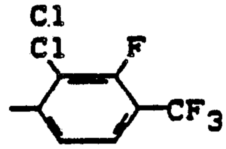 |
| $NO_2$ | H | 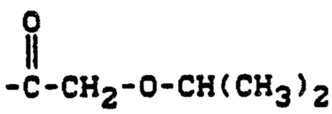 | 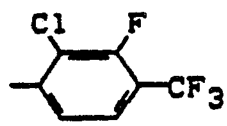 |
| $NO_2$ | H | 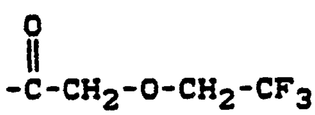 | 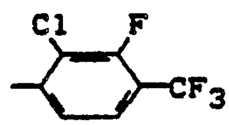 |
| $NO_2$ | H | 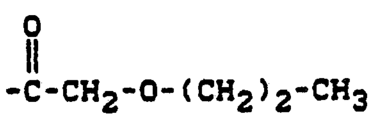 | 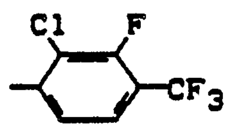 |

| $R^1$ | $R^2$ | $R^3$ | Ar |
| --- | --- | --- | --- |
| $NO_2$ | H | 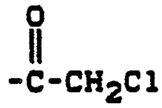 | 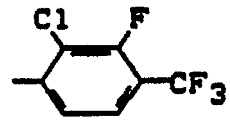 |
| $NO_2$ | H | 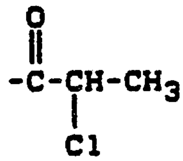 | 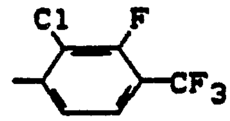 |
| $NO_2$ | H | 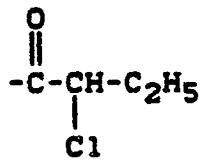 | 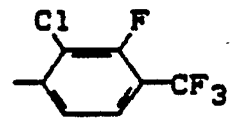 |
| $NO_2$ | H | 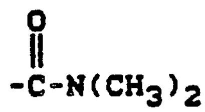 | 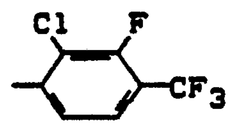 |
| $NO_2$ | H | 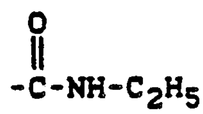 | 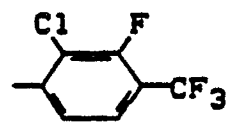 |
| $NO_2$ | H | $-SO_2-CH_3$ | 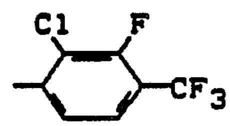 |
| $NO_2$ | H | 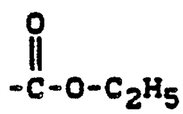 | 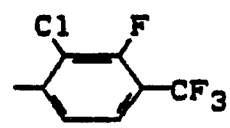 |
| $NO_2$ | H | 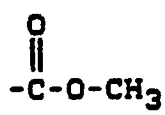 | 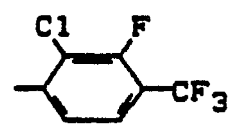 |

| $R^1$ | $R^2$ | $R^3$ | Ar |
|---|---|---|---|
| $NO_2$ | H | 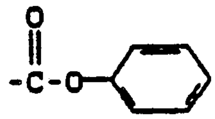 | 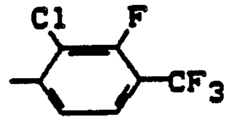 |
| $NO_2$ | H | 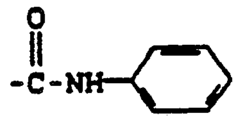 | 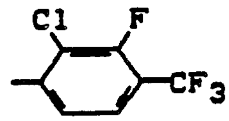 |
| $NO_2$ | H | $-\overset{O}{\overset{\|\|}{C}}-N(C_2H_5)_2$ | 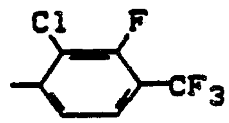 |
| $NO_2$ | H | $-\overset{O}{\overset{\|\|}{C}}-CH_2-OCH_3$ | 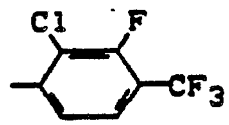 |
| $NO_2$ | H | $-\overset{O}{\overset{\|\|}{C}}-CH_2-O-C_2H_5$ | 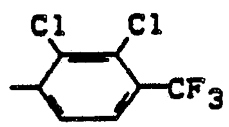 |
| $NO_2$ | H | $-\overset{O}{\overset{\|\|}{C}}-CH_2-O-CH(CH_3)_2$ | 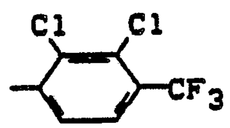 |
| $NO_2$ | H | $-\overset{O}{\overset{\|\|}{C}}-CH_2-O-CH_2-CF_3$ | 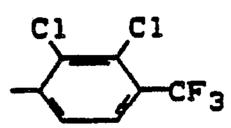 |
| $NO_2$ | H | $-\overset{O}{\overset{\|\|}{C}}-CH_2-O-(CH_2)_2-CH_3$ | 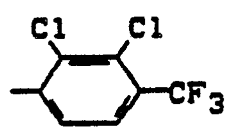 |
| $NO_2$ | H | $-\overset{O}{\overset{\|\|}{C}}-CH_2Cl$ | 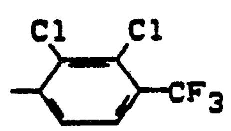 |

| $R^1$ | $R^2$ | $R^3$ | Ar |
|---|---|---|---|
| $NO_2$ | H | 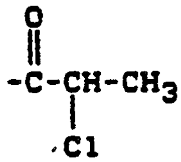 | 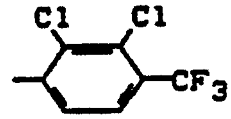 |
| $NO_2$ | H | 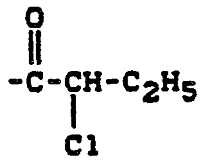 | 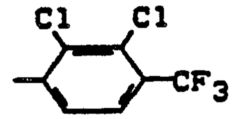 |
| $NO_2$ | H | 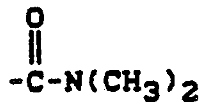 | 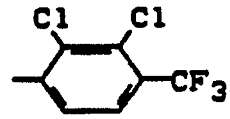 |
| $NO_2$ | H | 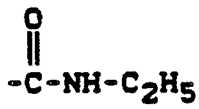 | 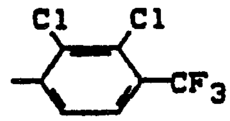 |
| $NO_2$ | H | $-SO_2-CH_3$ | 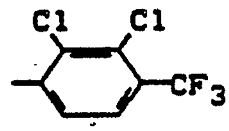 |
| $NO_2$ | H | 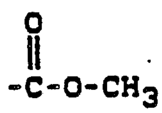 | 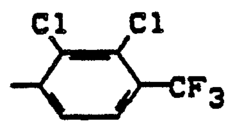 |
| $NO_2$ | H | 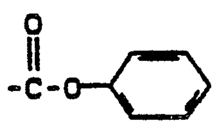 | 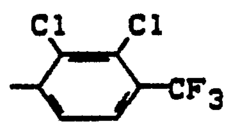 |
| $NO_2$ | H | 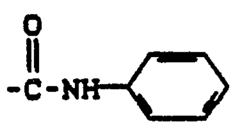 | 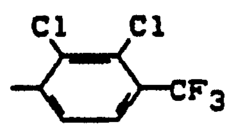 |
| $NO_2$ | H | 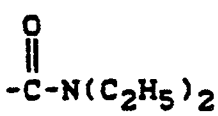 | 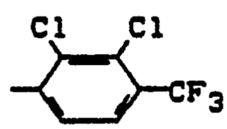 |

| $R^1$ | $R^2$ | $R^3$ | Ar |
|---|---|---|---|
| $NO_2$ | H | 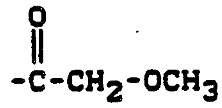 | 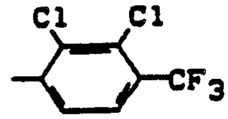 |
| $NO_2$ | H | 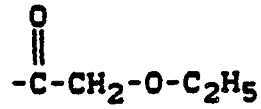 | 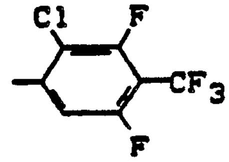 |
| $NO_2$ | H | 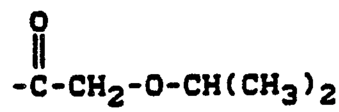 | 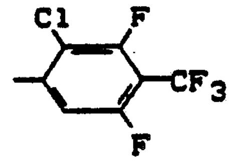 |
| $NO_2$ | H | 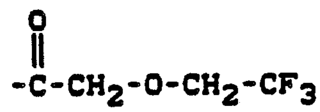 | 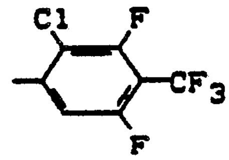 |
| $NO_2$ | H | 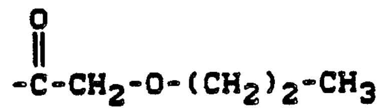 | 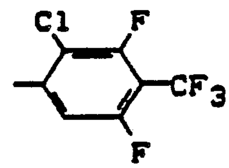 |
| $NO_2$ | H | 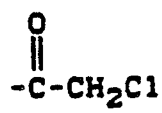 | 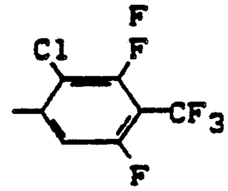 |
| $NO_2$ | H | 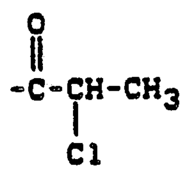 | 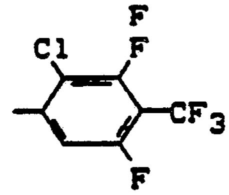 |
| $NO_2$ | H | 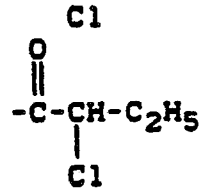 | 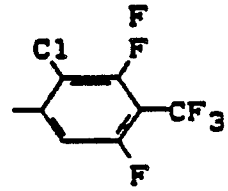 |
| $NO_2$ | H | 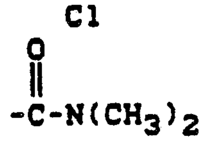 | 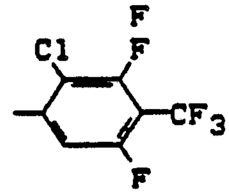 |

| $R^1$ | $R^2$ | $R^3$ | Ar |
|---|---|---|---|
| $NO_2$ | H | $-\overset{O}{\overset{\|}{C}}-NH-C_2H_5$ | 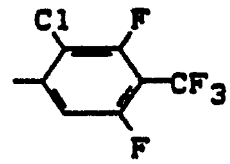 |
| $NO_2$ | H | $-SO_2-CH_3$ | 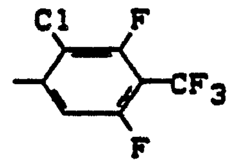 |
| $NO_2$ | H | $-\overset{O}{\overset{\|}{C}}-O-C_2H_5$ | 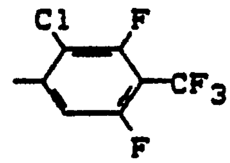 |
| $NO_2$ | H | $-\overset{O}{\overset{\|}{C}}-O-CH_3$ | 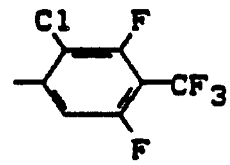 |
| $NO_2$ | H | 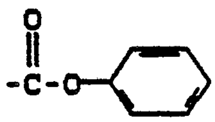 | 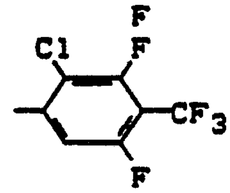 |
| $NO_2$ | H | 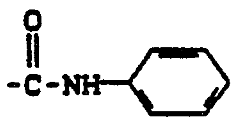 | 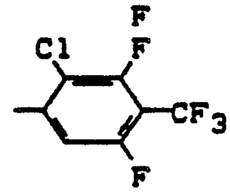 |
| $NO_2$ | H | $-\overset{O}{\overset{\|}{C}}-N(C_2H_5)_2$ | 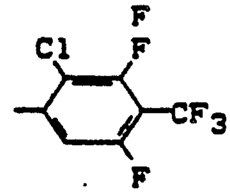 |
| $NO_2$ | H | $-\overset{O}{\overset{\|}{C}}-CH_2-OCH_3$ | 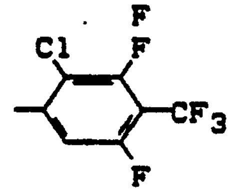 |
| $NO_2$ | H | $-\overset{O}{\overset{\|}{C}}-CH_2-O-C_2H_5$ | 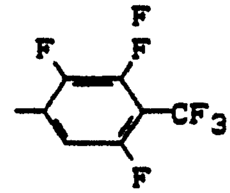 |

| $R^1$ | $R^2$ | $R^3$ | Ar |
|---|---|---|---|
| $NO_2$ | H | 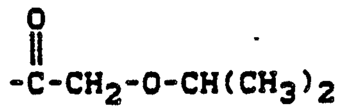 | 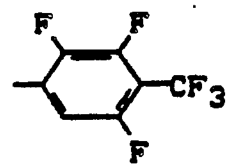 |
| $NO_2$ | H | 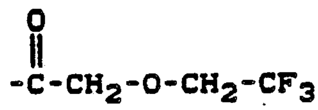 | 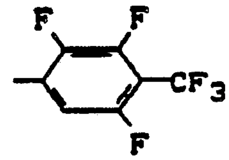 |
| $NO_2$ | H | 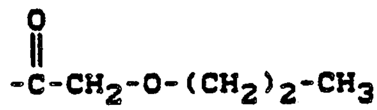 | 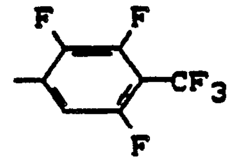 |
| $NO_2$ | H | 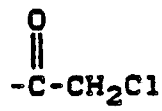 | 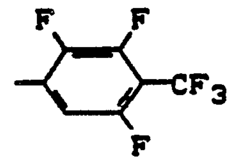 |
| $NO_2$ | H | 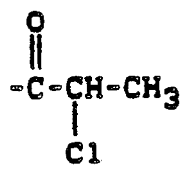 | 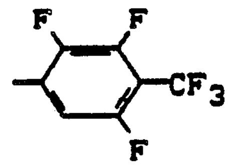 |
| $NO_2$ | H | 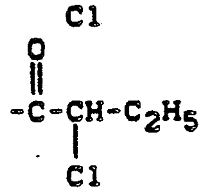 | 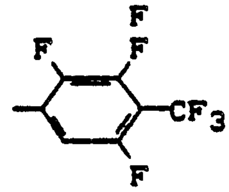 |
| $NO_2$ | H | 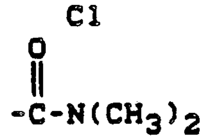 | 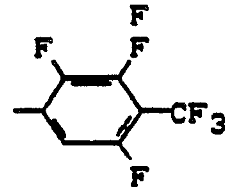 |
| $NO_2$ | H | 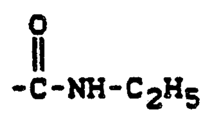 | 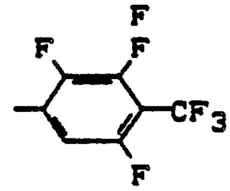 |
| $NO_2$ | H | $-SO_2-CH_3$ | 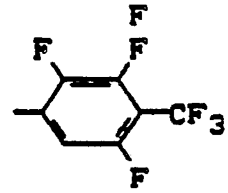 |

| $R^1$ | $R^2$ | $R^3$ | Ar |
|---|---|---|---|
| $NO_2$ | H | 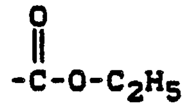 | 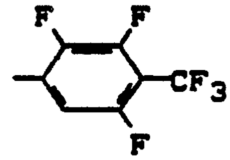 |
| $NO_2$ | H | 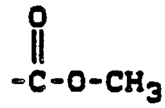 | 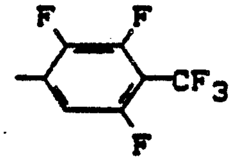 |
| $NO_2$ | H | 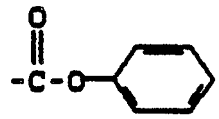 | 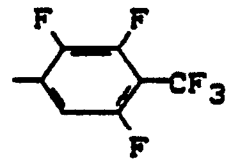 |
| $NO_2$ | H | 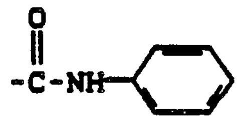 | 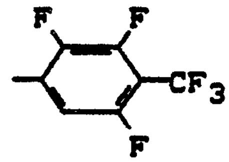 |
| $NO_2$ | H | 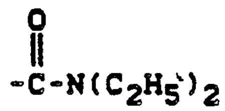 | 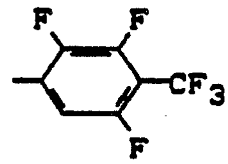 |
| $NO_2$ | H | 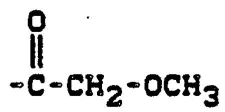 | 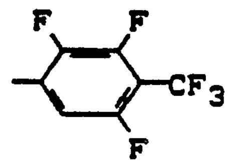 |

Verwendet man beispielsweise 5-Amino-1-(2-chlor-6-fluor-4-trifluormethylphenyl)-pyrazol und Acetanhydrid als Ausgangsstoffe, so läßt sich der Reaktionsablauf des erfindungsgemäßen Verfahrens (a-α) durch das folgende Formelschema darstellen:

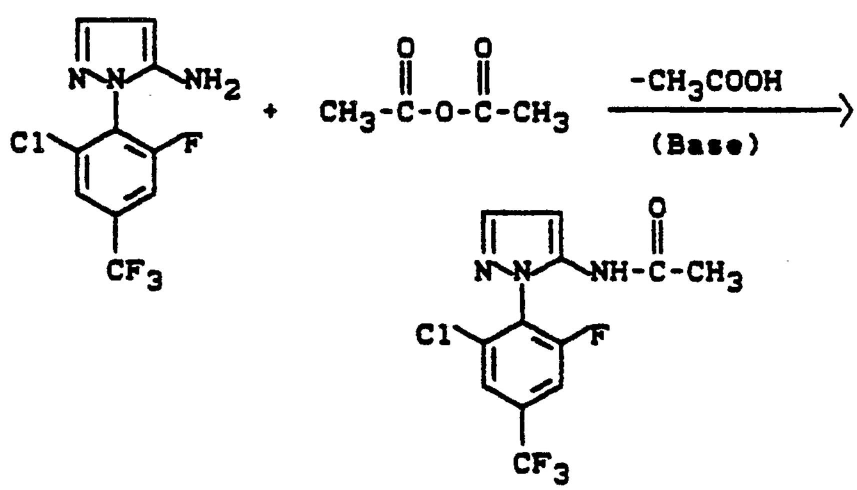


Verwendet man beispielsweise 5-Amino-1-(2,6-dichlor-3-fluor-4-trifluormethylphenyl)-pyrazol und Methansulfonsäurechlorid als Ausgangsstoffe, so läßt sich der Reaktionsablauf des erfindungsgemäßen Verfah-

rens, (a-β) durch das folgende Formelschema darstellen:

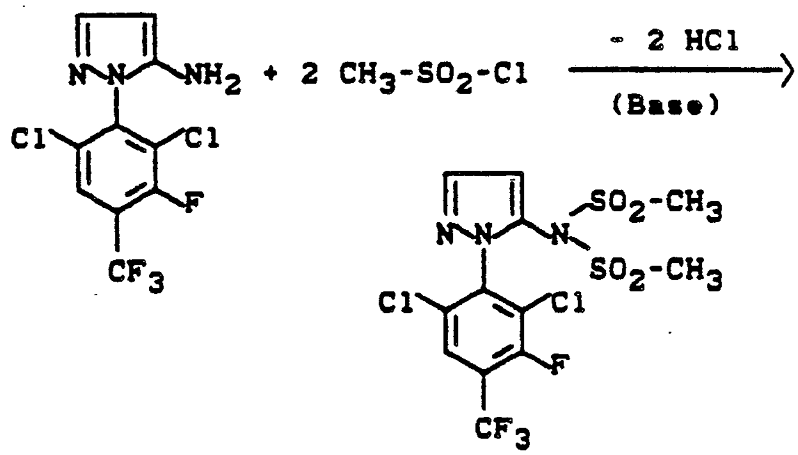


Verwendet man beispielsweise 4-Nitro-5-acetamido-1-(2,3-dichlor-4-trifluormethylphenyl)-pyrazol und Dimethylsulfat als Ausgangsstoffe, so läßt sich der Reaktionsablauf des erfindungsgemäßen Verfahrens (a-γ) durch das folgende Formelschema darstellen:

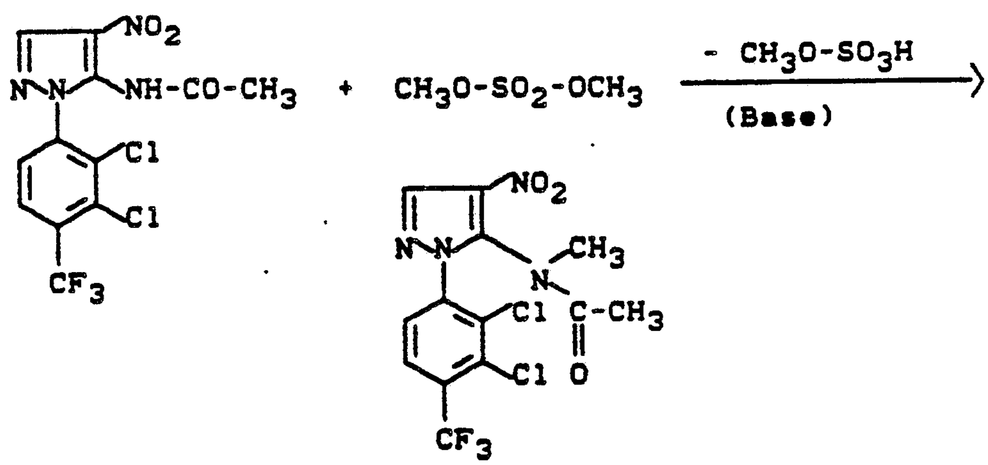


Verwendet man beispielsweise 5-Amino-4-nitro-1-(2,3,5-trifluor-4-trifluormethylphenyl)-pyrazol und Phenylisocyanat als Ausgangsstoffe, so läßt sich der Reaktionsablauf des erfindungsgemäßen Verfahrens (b) durch das folgende Formelschema darstellen:

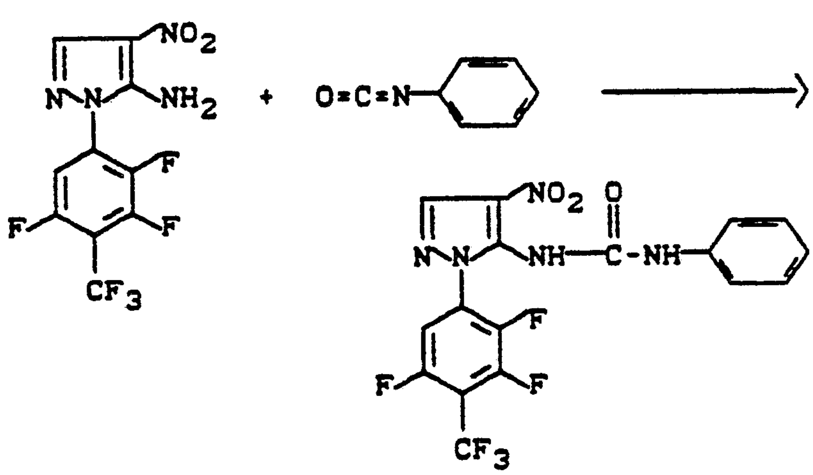

Verwendet man beispielsweise 5-Acetamido-1-(2-chlor-6-fluor-4-trifluormethylphenyl)-pyrazol und Salpetersäure als Ausgangsstoffe, so läßt sich der Reaktionsablauf des erfindungsgemäßen Verfahrens (c) durch das folgende Formelschema darstellen:

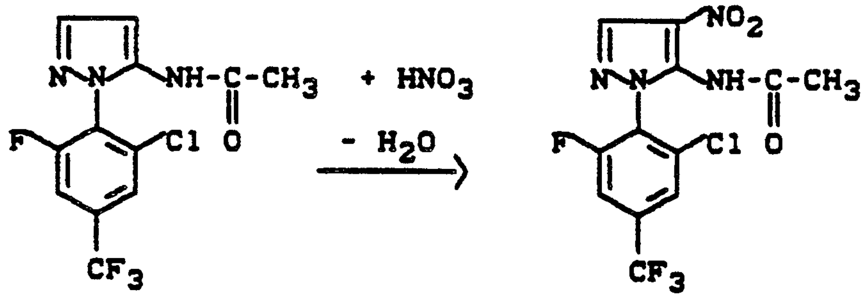


Verwendet man beispielsweise 5-(N-Ethyl-N-propionylamino)-4-nitro-1-(2-chlor-3,5-difluor-4-trifluormethylphenyl)-pyrazol als Ausgangsverbindung, so läßt sich der Reaktionsablauf des erfindungsgemäßen Verfahrens (d) durch das folgende Formelschema darstellen:

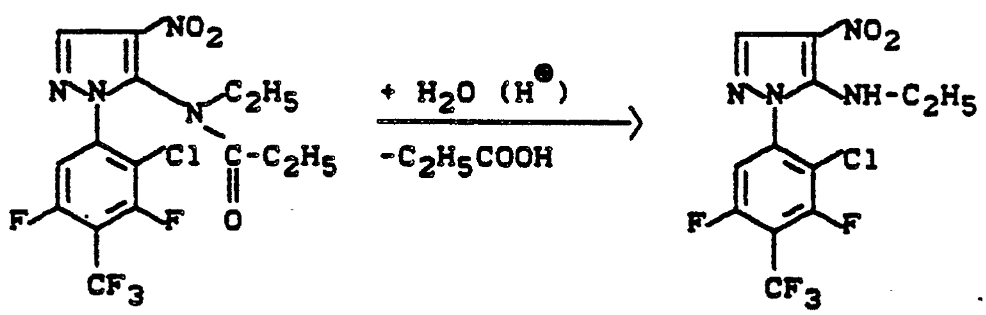


Verwendet man beispielsweise 5-[N,N-Bis-(methylsulfonyl)-amino]-1-(2-chlor-3-fluor-4-trifluormethylphenyl)-pyrazol und Ammoniak als Ausgangsstoffe, so läßt sich der Reaktionsablauf des erfindungsgemäßen Verfahrens (e) durch das folgende Formelschema darstellen:

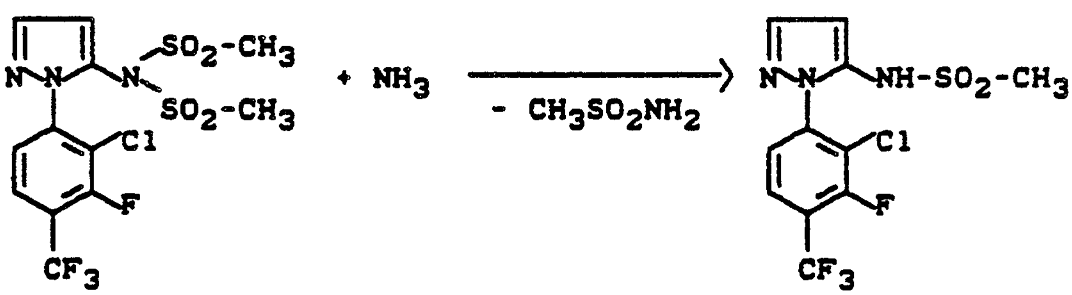


Verwendet man beispielsweise 5-Phenoxycarbonylamino-1-(2,3-difluor-6-chlor-4-trifluormethylphenyl)-pyrazol und Methanol als Ausgangsstoffe, so läßt sich der Reaktionsablauf des erfindungsgemäßen Verfahrens (f) durch das folgende Formelschema darstellen:

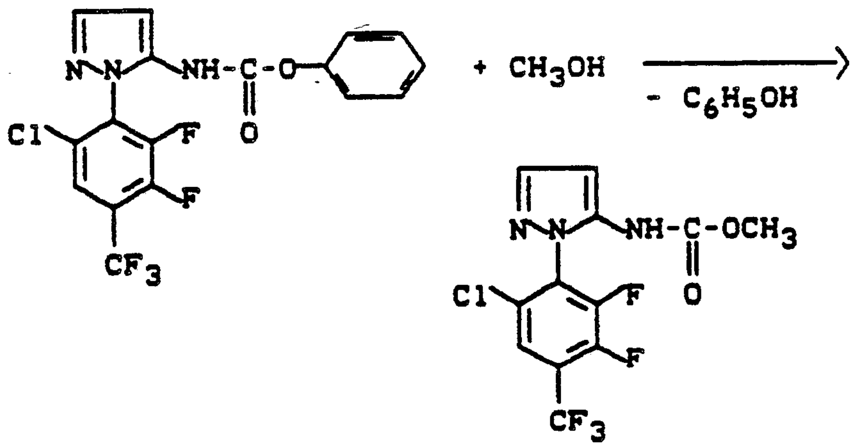


Verwendet man beispielsweise 5-Brom-4-nitro-1-(2,6-dichlor-3-fluor-4-trifluormethylphenyl)-pyrazol und Isopropylamin als Ausgangsstoffe, so läßt sich der Reaktionsablauf des erfindungsgemäßen Verfahrens (g) durch das folgende Formelschema darstellen:

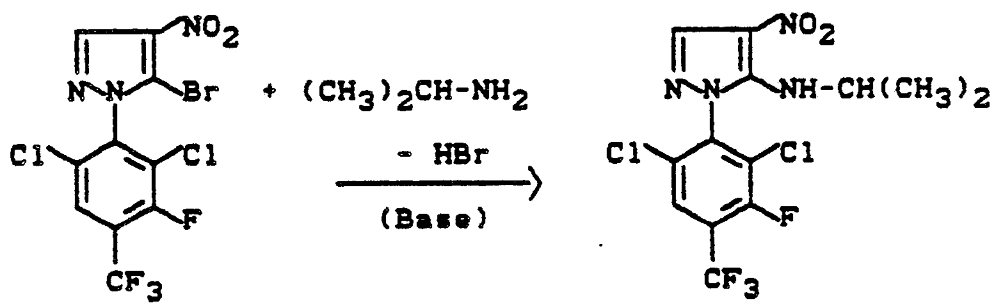


Verwendet man beispielsweise 4-Nitro-5-methansulfonamido-1-(2-chlor-6-fluor-4-trifluormethylphenyl)-pyrazol und Natriumhydrogencarbonat als Ausgangsstoffe, so läßt sich der Reaktionsablauf des erfindungsgemäßen Verfahrens (h) durch das folgende Formelschema darstellen:

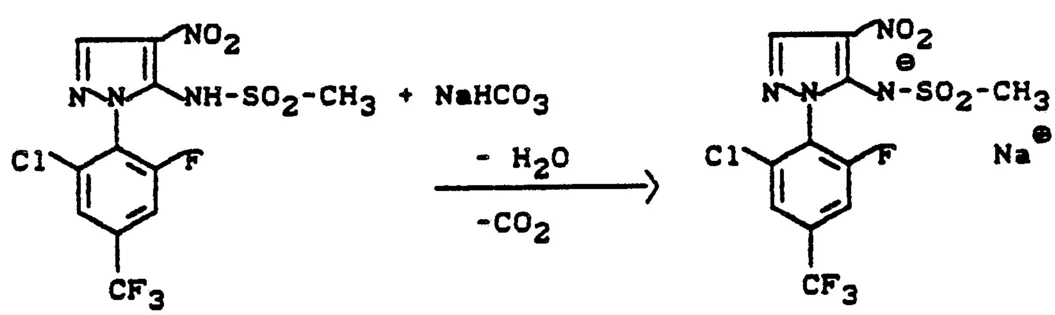


Die zur Durchführung der erfindungsgemäßen Verfahren (a) und (b) als Ausgangsstoffe benötigten 1-Arylpyrazole sind durch die Formel (II) allgemein definiert. In dieser Formel (II) stehen $R^1$, $R^2$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 1-Arylpyrazole der Formel (II) sind noch nicht bekannt.

1-Arylpyrazole der Formel (II), bei welchen $R^2$ für einen Rest $-\underset{\underset{X}{\|}}{C}-R^4$ oder für einen Rest $-S(O)_n-R^5$ steht, wobei $R^4$, $R^5$, X und n die oben angegebene Bedeutung haben, sind erfindungsgemäße Verbindungen und erhältlich mit Hilfe der erfindungsgemäßen Verfahren (a-α), (a-β), (b), (c), (e) oder (f).

1-Arylpyrazole der Formel (II), bei welchen $R^2$ für Wasserstoff steht sind Gegenstand einer eigenen

noch nicht publizierten Patentanmeldung (vergl. Deutsche Patentanmeldung P 36 17 977 vom 28.5.1986).

Man erhält sie, wenn man Arylhalogenide der Formel (XI),

Ar - $Hal^2$ (XI)

in welcher
$Hal^2$ für Halogen, insbesondere für Chlor oder Fluor steht und
Ar die oben angegebene Bedeutung hat,
zunächst in einer ersten Stufe mit Hydrazinhydrat gegebenenfalls in Gegenwart eines Verdünnungsmittels, wie beispielsweise Ethanol oder Pyridin bei Temperaturen zwischen 20 °C und 120 °C umsetzt; dann die so erhältlichen Arylhydrazine der Formel (XII),

Ar-NH-$NH_2$ (XII)

in welcher
Ar die oben angegebene Bedeutung hat,
in einer 2. Stufe mit Alkoxymethylenmalonesternitrilen der Formel (XIII),

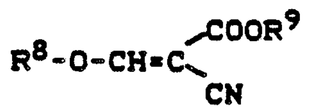

(XIII)

in welcher
$R^8$ und $R^9$ unabhängig voneinander jeweils für Alkyl, insbesondere für Methyl oder Ethyl stehen,
gegebenenfalls in Gegenwart eines Verdünnungsmittels wie beispielsweise Ethanol bei Temperaturen zwischen 20 °C und 120 °C umsetzt; dann die so erhältlichen 1-Arylpyrazol-4-carbonsäureester der Formel (XIV),

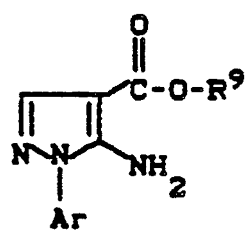

(XIV)

in welcher
$R^9$ und Ar die oben angegebene Bedeutung haben.
in einer 3.Stufe mit Säuren wie beispielsweise wässriger Schwefelsäure bei Temperaturen zwischen 80 °C und 150 °C verseift und decarboxyliert und die so erhältlichen 1-Aryl-5-amino-pyrazole der Formel (IIa),

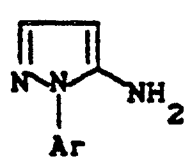

(IIa)

in welcher
Ar die oben angegebene Bedeutung hat,
gegebenenfalls in einer 4.Stufe mit einem Nitrierungsmittel wie beispielsweise Salpetersäure gegebenenfalls in Gegenwart eines Verdünnungsmittels wie beispielsweise Eisessig sowie gegebenenfalls in Gegenwart eines Reaktionshilfmittels wie beispielsweise Acetanhydrid, in Analogie zur Durchführung des erfindungsgemäßen Verfahrens (c), bei Temperaturen zwischen -20 °C und +50 °C nitriert.

Dabei kann es gegebenenfalls von Vorteil sein, vor der Nitrierungsreaktion die Aminogruppe in der 5-Position des Pyrazolringes mit Hilfe üblicher Schutzgruppentechniken, beispielsweise durch Acylierung zu schützen und die Aminoschutzgruppe nach erfolgter Nitrierung ebenfalls in üblicher Art und Weise beispielsweise durch Verseifung mit wäßriger oder alkoholischer Base wieder abzuspalten.

1-Aryl-5-aminopyrazole der Formel (IIa),

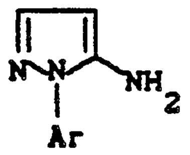

(IIa)

in welcher
Ar die oben angegebene Bedeutung hat,
erhält man alternativ auch, wenn man Arylhydrazine der Formel (XII),

$Ar-NH-NH_2$ (XII)

in welcher
Ar die oben angegebene Bedeutung hat,
mit 2-Halogenacrylnitril-Derivaten der Formel (XV),

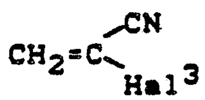

(XV)

in welcher
$Hal^3$ für Halogen, insbesondere für Chlor oder Brom steht,
gegebenenfalls in Gegenwart eines Verdünnungsmittels wie beispielsweise Ethanol sowie gegebenenfalls in Gegenwart eines Reaktionshilfsmittels wie beispielsweise Schwefelsäure oder Trifluoressigsäure bei Temperaturen zwischen 50 °C und 150 °C umsetzt.

Arylhalogenide der Formel (XI) sind größtenteils neu und Gegenstand einer parallel eingereichten Anmeldung. Man erhält sie in Analogie zu bekannten Verfahren (vergl. z.B. EP 187 023; EP 180 057; US 4 388 472; Zh. org. Khim. 20, 2187-2191 [1984] bzw. CA 102: 112944s; J. Fluorine Chem. 4, 317-326 [1974]; J.chem. Soc. C, 1969, 211-217).

Weiterhin wird in der oben erwähnten parallel eingereichten Anmeldung ein nicht-naheliegendes Verfahren zur Herstellung von mit Fluor und gegebenenfalls zusätzlich Chlor substituierten Benzotrifluoriden der Formel (XI) beschrieben, bei welchem Fluor und Chlor enthaltende Benzotrifluoride in Gegenwart eines Katalysators und eines Chlorwasserstoffakzeptors hydriert werden (vgl. die Herstellungsbeispiele (XI-3 bis XI-7).

Als Katalysatoren kommen im Prinzip bekannte Hydrierkatalysatoren in Frage, beispielsweise solche, welche die Elemente der VIII. Nebengruppe des Periodensystems in metallischer Form oder in Form von Verbindungen enthalten. Insbesondere geeignet sind Nickel, Platin, Palladium und deren Verbindungen, beispielsweise in Form von Raney-Nickel, metallischem Platin, metallischem Palladium und Palladium-tetra-(triphenylphosphin). Die katalytisch aktive Substanz kann auch auf Trägermaterialien aufgebracht sein, beispielsweise auf Aktivkohle, Siliziumdioxid, Aluminiumoxid, Silikaten oder Erdalkalisulfaten.

Bevorzugte Katalysatoren sind Raney-Nickel und metallisches Palladium auf Aktivkohle.

Die Katalysatoren können auch aus mehreren Komponenten zusammengesetzt sein und z.B. auch Promotoren enthalten, bei denen es sich auch um andere Elemente und Verbindungen als diejenigen der Metalle der VIII. Nebengruppe des Periodensystems handeln kann.

Die Menge des Katalysators ist im allgemeinen nicht kritisch. Sie kann beispielsweise 0,01 bis 15 Gew.-%, bezogen auf aktiven Katalysator betragen. Vorzugsweise beträgt diese Menge 0,1 bis 10 Gew.-% bezogen auf aktiven Katalysator (d.h. z.B. auf Pd, das auf Aktiv-Kohle aufgetragen ist.)

Die Hydrierung wird im allgemeinen in Gegenwart von Lösungs- oder Verdünnungsmitteln durchgeführt. Bei diesen ist es nicht zwingend erforderlich, daß sie die Einsatzmaterialien vollständig aufzulösen vermögen, da auch ein zweiphasiges Substrat hydriert werden kann. Beispielsweise können das Ausgangs-Benzotrifluorid und/oder der Chlorwasserstoff-Akzeptor während der Hydrierung ganz oder teilweise in suspendierter Form vorliegen. Geeignete Lösungs- und Verdünnungsmittel sind beispielsweise organische

Säuren, wie Essigsäure; Alkohole, wie Methanol, Ethanol, Propanol und Isopropanol; Ether, wie Tetrahydrofuran; Nitrile, wie Acetonitril und Wasser.

Als Chlorwasserstoff-Akzeptoren kommen die verschiedensten anorganischen und organischen Basen in Frage, beispielsweise die Hydroxide, Carbonate, Acetate und Ammoniumsalze der Alkali- und Erdalkalimetalle und Amine, insbesondere tertiäre Amine. Bevorzugt sind Natriumacetat und Triethylamin, N-N-Dimethylanilin, Pyrimidin und Picolin.

Der Chlorwasserstoff-Akzeptor kann in verschiedenen Mengen eingesetzt werden. Vorzugsweise werden pro Äquivalent aus dem eingesetzten Benzotrifluorid abzuspaltender Chloratome mindestens 0,8 Äquivalente Chlorwasserstoff-Akzeptor eingesetzt. Wenn aus dem eingesetzten Benzotrifluorid alle vorhandenen Chloratome abgespalten werden sollen ist die Menge des einzusetzenden Chlorwasserstoff-Akzeptors nach oben hin nicht kritisch. Aus praktischen Erwägungen ist es im allgemeinen vorteilhaft, in diesem Fall nicht mehr als 2 Äquivalente Chlorwasserstoff-Akzeptor pro Äquivalent abzuspaltender Chloratome einzusetzen. Wenn aus dem eingesetzten Benzotrifluorid nicht alle vorhandenen Chloratome abgespalten, d.h. noch Chloratome enthaltende Benzotrifluoride hergestellt werden sollen, so darf nicht wesentlich mehr Chlorwasserstoff-Akzeptor eingesetzt werden als stöchiometrisch erforderlich ist. Vorzugsweise setzt man in diesem Fall bis zu 1,2, besonders bevorzugt bis 1,05 und ganz besonders bevorzugt 1 Äquivalent Chlorwasserstoff-Akzeptor pro Äquivalent abzuspaltender Chloratome ein.

Die Hydrierung kann beispielsweise in Druckbreichen von Normaldruck bis 200 bar und bei Temperaturen im Bereich von 20 °C bis 200 °C durchgeführt werden. Bevorzugt sind Druckbereiche zwischen Normaldruck und 120 bar und Temperaturen im Bereich von 50 bis 140 °C.

Die Alkoxymethylenmalonesternitrile der Formel (XIII) und die Halogenacrylnitrilderivate der Formel (XV) sind allgemein bekannte Verbindungen der organischen Chemie.

Die zur Durchführung des erfindungsgemäßen Verfahrens (a-α) weiterhin als Ausgangsstoffe benötigten Acylierungsmittel sind durch die Formel (III) allgemein definiert. In dieser Formel (III) stehen $R^4$ und X vorzugsweise für diejenigen Reste, die bereits im Zusammenhang mit der Beschreibung der erfindungsgemäßen Stoffe der Formel (I) als bevorzugt für diese Substituenten genannt wurden.

$E^1$ steht vorzugsweise für Halogen, insbesondere für Chlor oder Brom oder für einen Rest $-O-\underset{\underset{O}{\|}}{C}-R^4$,

wobei $R^4$ die oben angegebene Bedeutung hat.

Die Acylierungsmittel der Formel (III) sind allgemein bekannte Verbindungen der organischen Chemie.

Die zur Durchführung des erfindungsgemäßen Verfahrens (a-α) weiterhin als Ausgangsstoffe benötigten Sulfenylierungs-, Sulfinylierungs- oder Sulfonylierungsmittel sind durch die Formel (IV) allgemein definiert. In dieser Formel (IV) stehen $R^5$ und n vorzugsweise für diejenigen Reste und Indices, die bereits im Zusammenhang mit der Beschreibung der erfindungsgemäßen Stoffe der Formel (I) als bevorzugt für diesen Substituenten und Index genannt wurden.

$E^2$ steht vorzugsweise für Halogen, insbesondere für Fluor, Chlor oder Brom.

Die Sulfenylierungs-, Sulfinylierungs- und Sulfonylierungsmittel sind ebenfalls allgemein bekannte Verbindungen der organischen Chemie.

Die zur Durchführung des erfindungsgemäßen Verfahrens (a-γ) weiterhin als Ausgangsstoffe benötigten Alkylierungsmittel sind durch die Formel (V) allgemein definiert. In dieser Formel (V) steht $R^{3-2}$ vorzugsweise für geradkettiges oder verzweigtes Alkyl mit 1 bis 6, insbesondere mit 1 bis 4 Kohlenstoffatomen.

$E^3$ steht vorzugsweise für Halogen, insbesondere für Brom oder Iod, für Alkoxysulfonyloxy oder für gegebenenfalls substituiertes Arylsulfonyloxy wie beispielsweise Methoxysulfonyloxy, Ethoxysulfonyloxy oder p-Toluolsulfonyloxy.

Die Alkylierungsmittel der Formel (V) sind ebenfalls allgemein bekannte Verbindungen der organischen Chemie.

Die zur Durchführung des erfindungsgemäßen Verfahrens (b) weiterhin als Ausgangsstoffe benötigten Iso(thio)cyanate sind durch die Formel (VI) allgemein definiert.

In dieser Formel (VI) steht X vorzugsweise für diejenigen Reste, die bereits im Zusammenhang mit der Beschreibung der erfindungsgemäßen Stoffe der Formel (I) als bevorzugt für diesen Substituenten genannt wurden.

$R^6$ steht vorzugsweise für geradkettiges oder verzweigtes Alkyl mit 1 bis 4 Kohlenstoffatomen oder für gegebenenfalls ein- bis dreifach, gleich oder verschieden substituiertes Phenyl, wobei als Substituenten in Frage kommen: Halogen, jeweils geradkettiges oder verzweigtes Alkyl oder Alkoxy mit jeweils 1 bis 4 Kohlenstoffatomen oder Halogenalkyl mit 1 bis 4 Kohlenstoffatomen und 1 bis 9 gleichen oder verschiedenen Halogenatomen. $R^6$ steht insbesondere für Methyl, Ethyl oder für gegebenenfalls ein- bis dreifach, gleich oder verschieden durch Fluor, Chlor, Methyl, Methoxy oder Trifluormethyl substituiertes Phenyl.

Die Iso(thio)cyanate der Formel (VI) sind ebenfalls allgemein bekannte Verbindungen der organischen Chemie.

Die zur Durchführung des erfindungsgemäßen Verfahrens (c) als Ausgangsstoffe benötigten 1-Arylpyrazole sind durch die Formel (Iz) allgemein definiert. In dieser Formel (Iz) stehen $R^2$, $R^3$, und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 1-Arylpyrazole der Formel (Iz) sind erfindungsgemäße Verbindungen und erhältlich mit Hilfe der erfindungsgemäßen Verfahren (a), (b), (d), (f) und (g).

Die zur Durchführung des erfindungsgemäßen Verfahrens (d) als Ausgangsstoffe benötigten 1-Arylpyrazole sind durch die Formel (Iy) allgemein definiert. In dieser Formel (Iy) stehen $R^1$, $R^4$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

$R^{3-2}$ steht vorzugsweise für geradkettiges oder verzweigtes Alkyl mit 1 bis 6, insbesondere mit 1 bis 4 Kohlenstoffatomen.

Die 1-Arylpyrazole der Formel (Iy) sind ebenfalls erfindungsgemäße Verbindungen und erhältlich mit Hilfe der erfindungsgemäßen Verfahren (a-γ) oder (c).

Die zur Durchführung des erfindungsgemäßen Verfahrens (e) als Ausgangsstoffe benötigten 1-Arylpyrazole sind durch die Formel (Ix) allgemein definiert. In dieser Formel (Ix) stehen $R^5$ und Ar vorzugsweise für diejenigen Reste, die bereits im Zusammenhang mit der Beschreibung der erfindungsgmäßen Stoffe der Formel (I) als bevorzugt für diese Substituenten genannt wurden.

Die 1-Arylpyrazole der Formel (Ix) sind ebenfalls erfindungsgemäße Verbindungen und erhältlich mit Hilfe des erfindungsgemäßen Verfahrens (a-β).

Die zur Durchführung des erfindungsgemäßen Verfahrens (f) als Ausgangsstoffe benötigten 1-Arylpyrazole sind durch die Formel (Iw) allgemein definiert. In dieser Formel (Iw) stehen $R^1$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

$R^7$ steht vorzugsweise für gegebenenfalls ein- bis dreifach durch Halogen und/oder niederes Alkyl substituiertes Phenyl, insbesondere für unsubstituiertes Phenyl,

$R^{2-1}$ steht vorzugsweise für Wasserstoff oder für einen Rest $-\underset{\underset{O}{\|}}{C}-O-R^7$,

wobei $R^7$ die oben angegebene Bedeutung hat.

Die 1-Arylpyrazole der Formel (Iw) sind ebenfalls erfindungsgemäße Verbindungen und erhältlich mit Hilfe der erfingungsgemäßen Verfahren (a-α) oder (c).

Die zur Durchführung des erfindungsgemäßen Verfahrens (f) weiterhin als Ausgangsstoffe benötigten Alkohole, Amine oder Thiole sind durch die Formel (VII) allgemein definiert. In dieser Formel (VII) steht $R^{4-1}$ vorzugsweise für jeweils geradkettiges oder verzweigtes Alkoxy, Alkylthio, Alkylamino oder Dialkylamino mit jeweils 1 bis 4 Kohlenstoffatomen in den einzelnen Alkylteilen oder für jeweils gegebenenfalls einfach oder mehrfach, gleich oder verschieden substituiertes Phenoxy, Phenylthio oder Phenylamino, wobei als Phenylsubstituenten jeweils in Frage kommen: Halogen, jeweils geradkettiges oder verzweigtes Alkyl oder Alkoxy mit jeweils 1 bis 4 Kohlenstoffatomen oder Halogenalkyl mit 1 bis 4 Kohlenstoffatomen und 1 bis 9 gleichen oder verschiedenen Halogenatomen, insbesondere Methyl, Methoxy, Chlor oder Trifluormethyl; $R^{4-1}$ steht insbesondere für Methoxy, Ethoxy, Methylthio, Phenylthio oder Dimethylamino.

Die Alkohole, Amine oder Thiole der Formel (VII) sind allgemein bekannte Verbindungen der organischen Chemie.

Die zur Durchführung des erfindungsgemäßen Verfahrens (g) als Ausgangsstoffe benötigten 5-Halogen-1-aryl-pyrazole sind durch die Formel (VIII) allgemein definiert. In dieser Formel (VIII) stehen $R^1$ und Ar vorzugsweise für diejenigen Reste, die bereits im Zusammenhang mit der Beschreibung der erfindungsgemäßen Stoffe der Formel (I) als bevorzugt für diese Substituenten genannt wurden. $Hal^1$ steht vorzugsweise für Chlor oder Brom.

Die 5-Halogen-1-aryl-pyrazole der Formel (VIII) sind noch nicht bekannt. Sie sind jedoch erhältlich in Analogie zu bekannten Verfahren (vergl. EP 191 282), beispielsweise wenn man Alkoxymethylenmalonester der Formel (XVI),

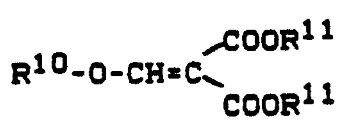

(XVI)

in welcher
$R^{10}$ und $R^{11}$ unabhängig voneinander jeweils für Alkyl, insbesondere für Methyl oder Ethyl stehen, mit Arylhydrazinen der Formel (XII),

Ar-NH-$NH_2$ (XII)

in welcher
Ar die oben angegebene Bedeutung hat,
zunächst in einer ersten Stufe gegebenenfalls in Gegenwart eines Verdünnungsmittels wie beispielsweise Methanol oder Ethanol bei Temperaturen zwischen +10 °C und +80 °C umsetzt, und die so erhältlichen Pyrazolcarbonsäureester der Formel (XVII),

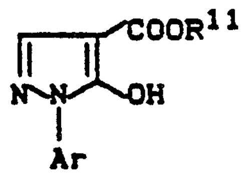

(XVII)

in welcher
$R^{11}$ und Ar die oben angegebene Bedeutung haben,
in einer 2.Stufe gegebenenfalls in Gegenwart eines Verdünnungsmittels wie beispielsweise Methanol und gegebenenfalls in Gegenwart einer Base wie beispielsweise Natriumhydroxid bei Temperaturen zwischen +30 °C und +70 °C hydrolysiert und decarboxyliert zu Pyrazolinonen der Formel (XVIII),

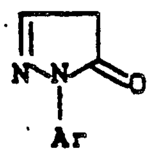

(XVIII)

in welcher
Ar die oben angegebene Bedeutung hat,
und diese in einer 3.Stufe mit Halogenierungsmitteln, wie beispielsweise Phosphoroxychlorid oder Phosphoroxybromid, nach üblichen bekannten Verfahren (vgl. z.B. Ber. dtsch. chem. Ges. 28, 35 (1895) oder Liebigs Ann. Chem. 373, 129 (1910)) umsetzt, und gegebenenfalls in einer 4-Stufe die so erhältlichen 5-Halogen-pyrazole der Formel (VIIIa),

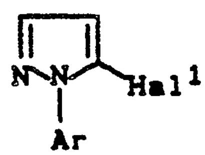

(VIIIa)

in welcher
$Hal^1$ und Ar die oben angegebene Bedeutung haben,
in Analogie zur Durchführung des erfindungsgemäßen Verfahrens (c) mit Salpetersäure gegebenenfalls in Gegenwart eines Verdünnungsmittels, wie beispielsweise Eisessig, und gegebenenfalls in Gegenwart eines Katalysators oder Reaktionshilfsmittels, wie beispielsweise Acetanhydrid bei Temperaturen zwischen -20 °C und +50 °C nitriert.

Die bei der Umsetzung von Alkoxymethylenmalonestern der Formel (XVI) mit Arylhydrazinen der Formel (XII) auftretenden Zwischenprodukte der Formel (XIX),

$$Ar-NH-NH-CH=C\begin{matrix}\diagup COOR^{11}\\ \diagdown COOR^{11}\end{matrix} \qquad (XIX)$$

in welcher
Ar und $R^{11}$ die oben angegebene Bedeutung haben,
können gegebenenfalls auch isoliert und in einer separaten Reaktionsstufe cyclisiert werden.

Die Cyclisierung zu den Pyrazolcarbonsäureestern der Formel (XVII) und deren anschließende Decarboxylierung können gegebenenfalls in einer Reaktionsstufe als "Eintopfverfahren" durchgeführt werden (vgl. z.B. Liebigs Ann. Chem. 373, 142 (1910)).

Die Alkoxymethylenmalonester der Formel (XVI) sind allgemein bekannte Verbindungen der organischen Chemie.

Die zur Durchführung des erfindungsgemäßen Verfahrens (g) weiterhin als Ausgangsstoffe benötigten Amine sind durch die Formel (IX) allgemein definiert. In dieser Formel (IX) steht $R^{3-2}$ vorzugsweise für geradkettiges oder verzweigtes Alkyl mit 1 bis 6 Kohlenstoffatomen, insbesondere für Methyl, Ethyl, n- oder i-Propyl oder n-, i-, s- oder t-Butyl.

Die Amine der Formel (IX) sind allgemein bekannte Verbindungen der organischen Chemie.

Die zur Durchführung des erfindungsgemäßen Verfahrens (h) als Ausgangsstoffe benötigten 1-Arylpyrazole sind durch die Formel (Iv) allgemein definiert. In dieser Formel (Iv) stehen $R^1$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

$R^{2-2}$ steht vorzugsweise für einen Rest $-SO_2-R^5$, wobei $R^5$ vorzugsweise diejenigen Reste steht, die bereits im Zusammenhang mit der Beschreibung der erfindungsgemäßen Stoffe der Formel (I) als bevorzugt für diesen Substituenten genannt wurden.

Die 1-Arylpyrazole der Formel (Iv) sind erfindungsgemäße Verbindungen und erhältlich mit Hilfe der erfindungsgemäßen Verfahren (a-$\alpha$), (a-$\beta$), (c), (e) oder (f).

Die zur Durchführung des erfingungsgemäßen Verfahrens (h) weiterhin als Ausgangsstoffe benötigten Salze sind durch die Formel (X) allgemein definiert. Vorzugsweise verwendet man Alkali-, Erdalkali-, Ammonium- oder Übergangsmetallhydroxide, -oxide, -carbonate, -hydrogencarbonate oder leicht lösliche -chloride, -sulfate, -phosphate oder -nitrate, wie beispielsweise Natrium-, Kalium- oder Calciumhydroxid, -carbonat oder -hydrogencarbonat, Calciumchlorid, Bariumchlorid, Kupfersulfat, Nickelchlorid oder Cobaltnitrat oder Alkylamine, wie Triethylamin, Isopropylamin, Diisopropylamin oder Butylamin.

Die Salze der Formel (X) sind allgemein bekannte Verbindungen der organischen Chemie.

Zur Durchführung des erfindungsgemäßen Verfahrens (a) kommen als Verdünnungsmittel inerte organische Lösungsmittel infrage. Vorzugsweise verwendet man aliphatische, alicyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Säuren, wie Essigsäure, Nitrile, wie Acetonitril oder Propionitril, Amide, wie Dimethyl formamid, Dimethylacetamid, N-Methylpyrrolidon oder Hexamethylphosphorsäuretriamid. Verwendet man als Reaktionspartner in den Varianten (a-$\alpha$), (a-$\beta$) oder (a-$\gamma$) Verbindungen der Formeln (III), (IV) oder (V) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (a) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfingungsgemäßen Verfahrens (a) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (a) setzt man pro Mol an 1-Arylpyrazol der Formel (II) in den Varianten (a-$\alpha$), (a-$\beta$) oder (a-$\gamma$) im allgemeinen jeweils 1,0 bis 20,0 Mol, vorzugsweise jeweils 1,0 bis 15,0 Mol, an Acylierungsmittel der Formel (III) oder Sulfenylierungs- bzw. Sulfinylierungs-

bzw. Sulfonylierungsmittel der Formel (IV) oder Alkylierungsmittel der Formel (V) und gegebenenfalls 1,0 bis 3,0 Mol, vorzugsweise 1,0 bis 2,0 Mol an Reaktionshilfsmittel, ein. Die Reaktionsdurchführung, Aufarbeitung und Isolierung der Reaktionsprodukte der Formel (Ia) erfolgt nach üblichen Methoden.

Zur Durchführung des erfindungsgemäßen Verfahrens (b) kommen als Verdünnungsmittel inerte organische Lösungsmittel in Frage. Vorzugsweise verwendet man die bei Verfahren (a) genannten Verdünnungsmittel. Verwendet man Iso(thio)cyanate der Formel (VI) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (b) kommen tertiäre Amine, wie beispielsweise Triethylamin, N,N-Dimethylanilin, Pyridin, 4-(N,N-Dimethylamino)-pyridin, Diazabicyclooctan (DABCO), Diazabcyclononen (DBN) oder Diazabicycloundecen (DBU), in Frage.

Die Reaktionstemperaturen können bei der Durchführung des erfindungsgemäßen Verfahrens (b) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (b) setzt man pro Mol 1-Aryl-pyrazol der Formel (II) im allgemeinen 1,0 bis 20,0 Mol, vorzugsweise 1,0 bis 15,0 Mol an Iso(thio)cyanat der Formel (VI) und gegebenenfalls 1,0 bis 3,0 Mol, vorzugsweise 1,0 bis 2,0 Mol an Reaktionshilfsmittel ein. Die Reaktionsdurchführung, Aufarbeitung und Isolierung der Reaktionsprodukte der Formel (Ib) erfolgt nach üblichen Methoden.

Als Verdünnungsmittel zur Durchführung des erfindungsgemäßen Verfahrens (c) kommen alle üblicherweise für derartige Nitrierungen verwendbaren Lösungsmittel in Frage. Vorzugsweise verwendet man die als Reagenzien in Frage kommenden Säuren oder Gemische, wie beispielsweise Schwefelsäure, Salpetersäure oder Nitriersäure, gleichzeitig als Verdünnungsmittel. Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff, als Verdünnungsmittel in Frage.

Als Reaktionshilfsmittel zur Durchführung des erfindungsgemäßen Verfahrens (c) kommen ebenfalls die für derartige Nitrierungen üblichen Katalysatoren in Frage; vorzugsweise verwendet man saure Katalysatoren wie beispielsweise Schwefelsäure oder Acetanhydrid.

Die Reaktionstemperaturen können bei der Durchführung der erfindungsgemäßen Verfahrens (c) in einem größeren Bereich variiert werden. Im allgemeinen arbeitet man zwischen -50 °C und +200 °C, vorzugsweise zwischen -20 °C und +150 °C.

Zur Durchführung des erfindungsgemäßen Verfahrens (c) setzt man pro Mol 1-Aryl-pyrazol der Formel (Iz), im allgemeinen 1,0 bis 10,0 Mol, vorzugsweise 1,0 bis 5,0 Mol an Salpetersäure und gegebenenfalls 0,1 bis 10 Mol an Reaktionshilfsmittel ein. Die Reaktionsführung, Aufarbeitung und Isolierung der Reaktionsprodukte der Formel (Ic) erfolgt in allgemein üblicher Art und Weise.

Als Verdünnungsmittel zur Durchführung des erfindungsgemäßen Verfahrens (d) kommen anorganische oder organische polare Lösungsmittel in Frage. Vorzugsweise verwendet man Alkohole, wie beispielsweise Methanol, Ethanol oder Propanol, oder deren Gemische mit Wasser.

Als Reaktionshilfsmittel zur Durchführung des erfindungsgemäßen Verfahrens (d) kommen vorzugsweise Säuren, insbesondere Chlorwasserstoffsäure oder Schwefelsäure in Frage.

Die Reaktionstemperaturen können bei der Durchführung des erfindungsgemäßen Verfahrens (d) in einem größeren Bereich variiert werden. Im allgemeinen arbeitet man zwischen +20 °C und +150 °C, vorzugsweise zwischen +50 °C und +120 °C.

Zur Durchführung des erfingungsgemäßen Verfahrens (d) setzt man pro Mol 1-Aryl-pyrazol der Formel (Iy) im allgemeinen 1,0 bis 20,0 Mol, vorzugsweise 1,0 bis 10,0 Mol an Katalysatorsäure ein und erwärmt für mehrere Stunden auf die erforderliche Reaktionstemperatur. Die Aufarbeitung, Isolierung und Reinigung der Reaktionsprodukte der Formel (Id) erfolgt nach üblichen Methoden.

Als Verdünnungsmittel zur Durchführung der erfindungsge mäßen Verfahrens (e) kommen polare organische Lösungsmittel oder deren Gemische mit Wasser in Frage.Vorzugsweise verwendet man Alkohole wie Methanol, Ethanol oder Propanol oder deren Gemische mit Wasser.

Als Reaktionshilfsmittel bei der Durchführung des erfindungsgemäßen Verfahrens (e) kommen alle üblichen anorganischen oder organischen Basen in Frage. Vorzugsweise verwendet man Amine oder Ammoniaklösungen oder Alkalimetallcarbonate bzw. -hydrogencarbonate, wie Natrium- oder Kaliumcarbonat oder Natriumhydrogencarbonat.

Die Reaktionstemperaturen können bei der Durchführung des erfindungsgemäßen Verfahrens (e) in einem größeren Bereich variiert werden. Im allgemeinen arbeitet man zwischen 0 °C und 80 °C, vorzugsweise zwischen 20 °C und 40 °C.

Zur Durchführung des erfindungsgemäßen Verfahrens (e) setzt man pro Mol 1-Aryl-pyrazol der Formel (Ix) im allgemeinen 1,0 bis 30,0 Mol vorzugsweise 1,0 bis 15,0 Mol an Base ein.

Die Reaktionsmischung wird in einem geeigneten Verdünnungsmittel so lange gerüht (30 Minuten bis 20 Stunden) bis bei chromatographischer Kontrolle kein Ausgangsprodukt mehr nachweisbar ist. Die Aufarbeitung und Isolierung der Reaktionsprodukte der Formel (Ie) erfolgt nach üblichen Methoden.

Als Verdünnungsmittel zur Durchführung der erfindungsgemäßen Verfahren (f) kommen inerte organische Lösungsmit tel in Frage. Vorzugsweise verwendet man aliphatische, oder aromatische, gegebenenfalls halogenierte Kohlenwasserstoffe, wie Benzin, Benzol, Toluol, Xylol, Pentan, Hexan, Heptan, Cyclohexan, Petrolether, Ligroin, Methylenchlorid, Ethylenchlorid, Chloroform, Tetrachlorkohlenstoff,Chlorbenzol oder Dichlorbenzol, Ether, wie Diethylether oder Diisopropylether; Ethylenglykoldimethylether, Tetrahydrofuran oder Dioxan, Ketone, wie Aceton oder Butanon, Methylisopropylketon oder Methylisobutylketon, Ester, wie Essigsäureethylester, Nitrile, wie Acetonitril oder Propionitril, Amide, wie Dimethylformamid, Dimethylacetamid, N-Methylpyrrolidon oder Hexamethylphosphorsäuretriamid, oder Alkohole wie Methanol, Ethanol oder Isopropanol.

Es ist jedoch auch möglich, die als Reaktionskomponenten verwendeten Alkohole, Amine oder Thiole der Formel (VII) in entsprechendem Überschuß gleichzeitig als Verdünnungsmittel einzusetzen.

Das erfindungsgemäße Verfahren (f) kann gegebenenfalls in Gegenwart eines basischen Reaktionshilfsmittel durchgeführt werden. Als solche kommen alle üblichen anorganischen oder organischen Basen infrage. Vorzugsweise verwendet man Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei dem erfindungsgemäßen Verfahren (f) ebenfalls in einem größeren Bereich variiert werden. Im allgemeinen arbeitet man zwischen 0 °C und +200 °C, vorzugsweise zwischen +20 °C und +150 °C.

Zur Durchführung des erfindungsgemäßen Verfahrens (f) setzt man pro Mol 1-Arylpyrazol der Formel (Iw) im allgemeinen 1 bis 20 Mol, vorzugsweise 1 bis 10 Mol an Al kohol, Amin oder Thiol der Formel (VII) und gegebenenfalls 0,1 bis 2 Mol, vorzugsweise 0,1 bis 1 Mol an Reaktionshilfsmittel ein und erwärmt für mehrere Stunden auf die erforderliche Temperatur. Die Aufarbeitung und Isolierung der Reaktionsprodukte der Formel (If) erfolgt nach üblichen Verfahren.

Als Verdünnungsmittel zur Durchführung des erfindungsgemäßen Verfahrens (g) kommen inerte organische Lösungsmittel in Frage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (g) kann gegebenenfalls in Gegenwart eines geeigneten Reaktionshilfsmittels durchgeführt werden.

Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Es ist jedoch auch möglich, einen entsprechenden Überschuß an dem als Reaktionspartner eingesetzten Amin der Formel (IX) gleichzeitig als Reaktionshilfsmittel zu verwenden.

Die Reaktionstemperaturen können bei der Durchführung des erfindungsgemäßen Verfahrens (g) in einem größeren Bereich variiert werden. Im allgemeinen arbeitet man bei Temperaturen zwischen -20 °C und +200 °C, vorzugsweise bei Temperaturen zwischen 0 °C und +150 °C.

Zur Durchführung des erfindungsgemäßen Verfahrens (g) setzt man pro Mol an 5-Halogen-1-arylpyrazol der Formel (VIII) im allgemeinen 1,0 bis 10,0 Mol, vorzugsweise 1,0 bis 5,0 Mol an Amin der Formel (IX) ein. Die Reaktionsdurchführung, Aufarbeitung und Isolierung der Reaktionsprodukte der Formel (Ig) erfolgt nach allgemein üblichen Verfahren.

Als Verdünnungsmittel zur Durchführung des erfindungsgemäßen Verfahrens (h) kommen polare organische Lösungsmittel, Wasser oder wässrige Gemische, in Frage. Vorzugsweise verwendet man Alkohole, wie beispielsweise Methanol, Ethanol oder Propanol, deren Gemische mit Wasser oder reines Wasser.

Die Reaktionstemperaturen können bei der Durchführung des erfindungsgemäßen Verfahrens (h) ebenfalls in einem größeren Bereich variiert werden. Im allgemeinen arbeitet man zwischen 0 °C und +80 °C, vorzugsweise zwischen +20 °C und +40 °C.

Zur Durchführung des erfindungsgemäßen Verfahrens (h) setzt man pro Mol an 1-Aryl-pyrazol der Formel (Iv) im allgemeinen 1,0 bis 10 Mol, vorzugsweise 1,0 bis 5,0 Mol an Salz der Formel (X) oder an Amin ein. Zur Herstellung der Natrium-, Kalium- oder Ammoniumsalze setzt man eine Verbindung der Formel (Iv) in wässriger Lösung oder einem organischen Lösungsmittel, wie Aceton, Methanol, Ethanol oder Dimethylformamid, mit Natrium-, Kalium- oder Ammoniumhydroxid oder einem Amin um und isoliert die Salze durch Abfiltrieren oder durch Eindampfen der Lösung und reinigt sie gegebenenfalls durch Umkristallisieren.

Die Calcium-, Barium-, Magnesium-, Mangan-, Kupfer-, Nickel-, Zinn-, Eisen- oder Cobaltsalze werden hergestellt aus den Natriumsalzen durch Behandeln mit einem entsprechenden anorganischen Metallsalz, z.B. Calcium chlorid, Bariumchlorid, Kupfersulfat, Nickelchlorid oder Cobaltnitrat. Die Calciumsalze können auch hergestellt werden durch Behandeln einer Verbindung der Formel (Iv) mit Calciumhydroxid.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotina, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-. Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung mono-und dikotyler Unkräuter in monokotylen und dikotylen Kulturen wie beispielsweise Weizen, Gerste, Roggen, Reis, Mais, Baumwolle oder Sojabohnen einsetzen.

Auch die Vorprodukte der Formel (VIII) besitzen eine herbizide Wirksamkeit.

In entsprechenden Aufwandmengen zeigen die erfindungsgemäßen Wirkstoffe außerdem eine breite

fungizide Wirksamkeit und lassen sich beispielsweise zur Bekämpfung von Schorfpilzen (Venturia Arten) oder zur Bekämpfung von echten Mehltaupilzen im Obst- und Gemüseanbau sowie zur Bekämpfung von Reiskrankheiten wie beispielsweise gegen den Erreger der Reisfleckenkrankheit (Pyricularia oryzae) einsetzen.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polaren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine,z.B. Erdölfraktionen, mineralische und pflanzliche Öle, Alkohole wie Butanol oder Glycol sowie der Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablauge und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N′-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit 5-(2-Chlor-4-trifluormethyl-phenoxy)-2-nitro-benzoesäure; Chloressigsäure-N-(methoxy methyl)-2,6-diethylanilid; Methyl-6,6-dimethyl-2,4-dioxo-3-[1-(2-propenyloxyamino)-butyliden]-cyclohexancarbonsäure; 2-Chlor-4-ethylamino-6-isopropylamino-1,3,5-triazin; 3-Isopropyl-2,1,3-benzothiadiazin-4-on-2,2-dioxid; Methyl-5-(2,4-dichlorphenoxy)-2-nitrobenzoat; 3,5-Dibrom-4-hydroxy-benzonitril; 2-Chlor-N-{[(4-methoxy-6-methyl-1,3,5-triazin-2-yl)-amino]-carbonyl}-benzolsulfonamid; N,N-Dimethyl-N′-(3-chlor-4-methylphenyl)-harnstoff; exo-1-Methyl-4-(1-methylethyl)-2-(2-methylphenyl-methoxy)-7-oxabicyclo-(2,2,1)-heptan; Ethyl-2-{[(4-chlor-6-methoxy-2-pyrimidinyl)-aminocarbonyl]-aminosulfonyl}-benzoat; 2 Chlor-4-ethylamino-6-(3-cyanopropylamino)-1,3,5-triazin; N,N-Di-n-propylthiocarbamidsäure-S-ethylester; 4-Amino-6-t-butyl-3-ethylthio-1,2,4-triazin-5(4H)-on; 2-{4-[(6-Chlor-2-benzoxazolyl)-oxy]-phenoxy}-propansäure, deren Methyl-oder deren Ethylester; 2-[4-(3,5-Dichlorpyrid-2-yloxy)-phenoxy]-propionsäure-(trimethylsilylmethylester); 5-(2-Chlor-4-trifluormethyl-phenoxy)-N-methylsulfonyl-2-nitrobenzamid; N-Phosphonomethyl-glycin; 2-{4-[(3-Chlor-5-(trifluormethyl)-2-pyridinyl)-oxy]-phenoxy}-propansäure bzw. -propansäureethylester; 2-[4-(2,4-Dichlorphenoxy)-phenoxy]-propionsäuremethylester; Methyl-2-[4,5-dihydro-4-methyl-4-(1-methylethyl)-5-oxo-1H-imidazol-2-yl]-4(5)-methylbenzoat; 3,5-Diiod-4-hydroxybenzonitril; N,N-Dimethyl-N′-(4-isopropylphenyl)-harnstoff; (2-Methyl-4-chlorphenoxy)-essigsäure; (4-Chlor-2-methylphenoxy)-propionsäure; 2,4-Dichlorphenoxyessigsäure; 2,4-Dichlorphenoxypropionsäure; N-Methyl-2-(1,3-benzthiazol-2-yloxy)-acetanilid; 2-Chlor-N-(2,6-dimethylphenyl)-N-[(1H)-pyrazol-1-yl-methyl]-acetamid; 2-Ethyl-6-methyl- N-(1-methyl-2-methoxyethyl)-chloracetanilid; 2-{[[((4-Methoxy-6-methyl-1,3,5-triazin-2-yl)-amino)-carbonyl]-amino]-sulfonyl}-benzoesäure oder deren Methylester; N-(1-Ethylpropyl)-3,4-dimethyl-2,6-dinitroanilin; 3-(Ethoxycarbonylaminophenyl)-N-(3′-methylphenyl)-carbamat; α-Chlor-2′,6′-diethyl-N-(2-propoxyethyl)-acetanilid; 0-(6-Chlor-3-phenyl-pyridazin-4-yl)-S-octyl-thiocarbonat; 2-[1-(Ethoxamino)-butyliden]-5-(2-ethylthiopropyl)-1,3-cyclohexadion; 2-Chlor-4,6-bis-(ethylamino)-1,3,5-triazin; Methyl-2-{[(4,6-dimethyl-2-pyrimidinyl)-aminocarbonyl]-aminosulfonyl}-benzoat; 4-Ethylamino-2-t-butylamino-6-methylthio-s-triazin; N,N-Diisoproyl-S-(2,3,3-trichlorallyl)-thiolcarbamat oder 2,6-Dinitro-4-trifluormethyl-N,N-dipropylanilin sind gegebenenfalls von Vorteil. Einige Mischungen zeigen überraschenderweise auch synergistische Wirkung.

Auch Mischungen mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln sind möglich.

Die Wirkstoffe können als solche, in Form ihrer Formulierungen oder den daraus durch weeiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann bei der Anwendung der erfindungsgemäßen Verbindungen als Herbizide in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Bei der Anwendung der erfindungsgemäßen Verbindungen als Pflanzenwachtstumsregulatoren können die Aufwandmengen ebenfalls in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Herstellungsbeispiele:

Beispiel 1:

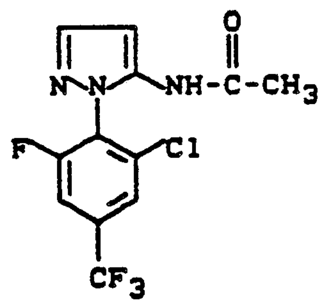

(Verfahren a-α)

Zu 100 g (0,036 Mol) 5-Amino-1-(2-chlor-6-fluor-4-trifluormethylphenyl)-pyrazol in 60 ml Eisessig gibt man 3,7 ml (0,038 Mol) Acetanhydrid und rührt 9 Stunden bei Raumtemperatur. Zur Aufarbeitung tropft man die Reaktionsmischung in 300 ml Eiswasser, filtriert den entstandenen Niederschlag ab, wäscht mit Wasser nach und trocknet im Vakuum bei 50 °C.
Man erhält 8,9 g (77 % der Theorie) an 5-Acetamido-1-(2-chlor-6-fluor-4-trifluormethylphenyl)-pyrazol vom Schmelzpunkt 144-146 °C.

Beispiel 2:

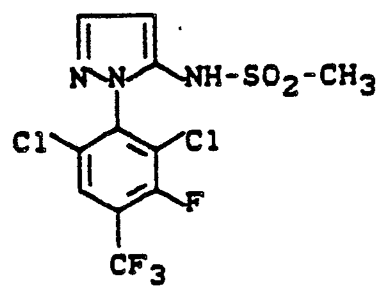

(Verfahren a-β/e)

Zu einer Lösung aus 6,3 g (0.02 Mol) 5-Amino-1-(2,6-dichlor-3-fluor-4-trifluormethyl-phenyl)-pyrazol in 30 ml wasserfreiem Pyridin gibt man bei 0 °C 3,2 ml (0.041 Mol) 99-prozentiges Methansulfonsäurechlorid. Es wird 16 Stunden bei Raumtemperatur gerührt und danach auf ca. 400 ml Eiswasser ausgetragen. Man versetzt mit 200 ml Dichlormethan, trennt die organische Phase ab, wäscht mit verdünnter Salzsäure und entfernt das Lösungsmittel im Vakuum. Der Rückstand wird in 75 ml Ethanol gelöst, mit 15 ml konzentrierter Ammoniaklösung versetzt und 24 Stunden bei 0 °C - 5 °C gerührt. Das Ethanol wird im Vakuum entfernt, der Rückstand mit Dichlormethan extrahiert, die organische Phase nacheinander mit verdünnter Salzsäure, gesättigter Natriumhydrogencarbonat- und gesättigter Kochsalzlösung gewaschen. Nach der Entfernung des Lösungsmittels im Vakuum erhält man 5,3 g (68 % der Theorie) an 5-Methansulfonamido-1-(2,6-dichlor-3-fluor-4-trifluormethylphenyl)-pyrazol vom Schmelzpunkt 141-143 °C.

Beispiel 3:

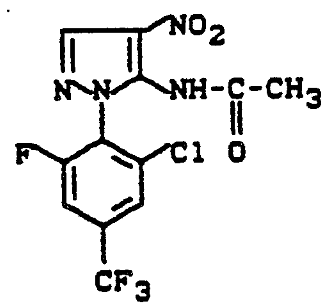

(Verfahren c)

Zu 6 g (0,019 Mol) 5-Acetamido-1-(2-chlor-6-fluor-4-trifluormethylphenyl)-pyrazol in 15 ml Eisessig gibt man bei Raumtemperatur nacheinander 2 ml (0,021 Mol) Acetanhydrid und 0,9 ml (0,02 Mol) 98%ige Salpetersäure und rührt 15 Stunden bei Raumtemperatur. Zur Aufarbeitung tropft man die Reaktionsmischung in 200 ml Eiswasser, filtriert den entstandenen Niederschlag ab, wäscht mit Wasser nach und trocknet im Vakuum bei 50 °C.
Man erhält 5,8 g (85 % der Theorie) an 5-Acetamido-4-nitro-1-(2-chlor-6-fluor-4-trifluormethylphenyl)-pyrazol vom Schmelzpunkt 129-130 °C.

Beispiel 4:

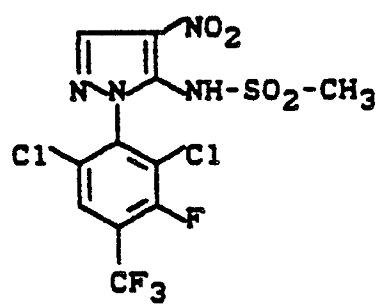

(Verfahren c)

Zu einer Lösung von 3,8 g (0.01 Mol) 5-Methansulfonamido-1-(2,6-dichlor-3-fluor-4-trifluormethylphenyl)-pyrazol in 20 ml Eisessig gibt man bei ca. 15 °C nacheinander 1,07 ml (0.011 Mol) Essigsäureanhydrid und 0,45 ml (0.0106 Mol) 98-prozentige Salpetersäure. Man rührt ca. 16 Stunden bei Raumtemperatur und trägt dann die Reaktionslösung in 100 ml Wasser aus. Der so erhaltene Niederschlag wird abgesaugt, neutral gewaschen und im Vakuum getrocknet. Man erhält 3,5 g (80 % der Theorie) an 5-Methansulfonamido-4-nitro-1-(2,6-dichlor-3-fluor-4-trifluormethylphenyl)-pyrazol vom Schmelzpunkt 107-115 °C.

In entsprechender Weise und gemäß den allgemeinen Angaben zur Herstellung erhält man die folgenden 1-Arylpyrazole der allgemeinen Formel (I):

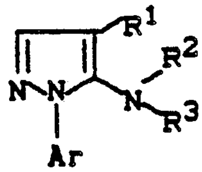

(I)

**Tabelle 1**

| Bsp. Nr. | $R^1$ | $R^2$ | $R^3$ | Ar | physikalische Eigenschaften |
|---|---|---|---|---|---|
| 5 | H | H | $-CO-C_2H_5$ | 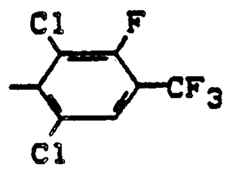 | Fp:75-85° C |
| 6 | $NO_2$ | H | $-CO-C_2H_5$ | 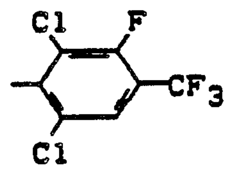 | Fp:77-80° C |
| 7 | H | H | $-CO-CH_3$ | 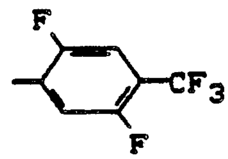 | Fp:109-111° C |
| 8 | $NO_2$ | H | $-CO-CH_3$ | 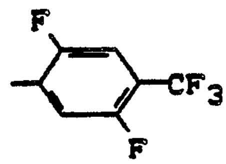 | Fp:158-160° C |

Tabelle 1 (Fortsetzung)

| Bsp. Nr. | $R^1$ | $R^2$ | $R^3$ | Ar | physikalische Eigenschaften |
|---|---|---|---|---|---|
| 9 | H | H | $-CO-CH_3$ | 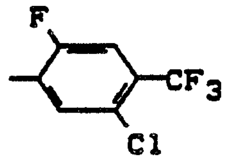 | Fp:87-90° C |
| 10 | H | H | $-CO-CH_3$ | 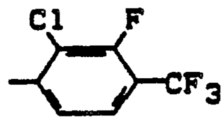 | Fp:126-128° C |
| 11 | $NO_2$ | H | $-CO-CH_3$ | 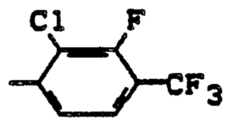 | Fp:73-77° C |
| 12 | H | H | $-CO-CH_3$ | 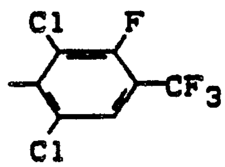 | Fp:146-149° C |
| 13 | $NO_2$ | H | $-CO-CH_3$ | 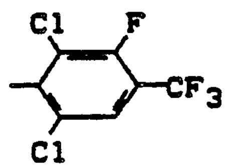 | Fp:67-70° C |
| 16 | H | H | -CO-H | 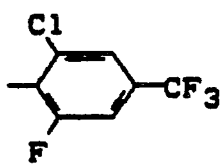 | Fp:93-109° C |
| 15 | H | H | $-CO-(CH_2)_2-CH_3$ | 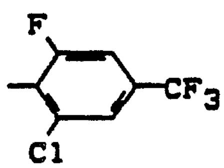 | Fp:84-86° C |
| 16 | H | H | $-SO_2-CH_3$ | 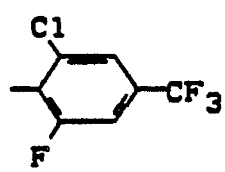 | $^1$H-NMR*) 2,95(s); 7,05(s); 7,45(dd) |

**T a b e l l e 1** (Fortsetzung)

| Bsp. Nr. | $R^1$ | $R^2$ | $R^3$ | Ar | physikalische Eigenschaften |
|---|---|---|---|---|---|
| 17 | H | H | 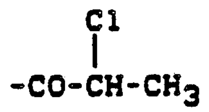 | 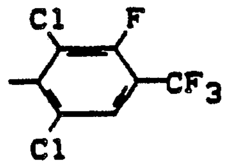 | Fp:74-79° C |
| 18 | H | H | $-CO-(CH_2)_2-CH_3$ | 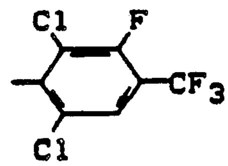 | Fp:117-120° C |
| 19 | H | H | $-CO-CH_2-OCH_3$ | 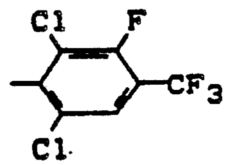 | Fp:91-93° C |
| 20 | H | H | -CO-H | 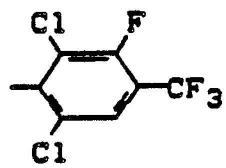 | Fp:89-107° C |
| 21 | H | H | $-CO-OCH_3$ | 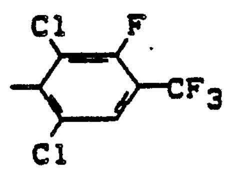 | Fp:69-76° C |
| 22 | H | H | $-CO-CH_2-OCH_3$ | 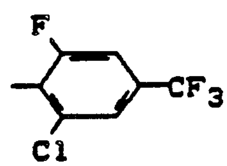 | Fp:96° C |
| 23 | H | H | $-CO-OCH_3$ | 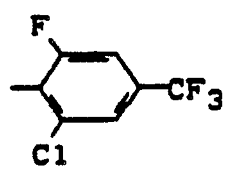 | Fp:103-105° C |
| 24 | $NO_2$ | H | 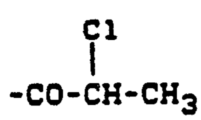 | 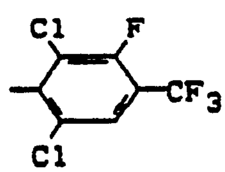 | Fp:79-81° C |

T a b e l l e 1 (Fortsetzung)

| Bsp. Nr. | $R^1$ | $R^2$ | $R^3$ | Ar | physikalische Eigenschaften |
|---|---|---|---|---|---|
| 25 | $NO_2$ | H | $-CO-(CH_2)_2-CH_3$ | 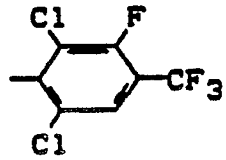 | Fp:66-68° C |
| 26 | $NO_2$ | H | $-CO-CH_2-OCH_3$ | 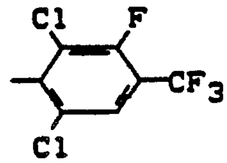 | Fp:85-87° C |
| 27 | $NO_2$ | H | $-SO_2-CH_3$ | 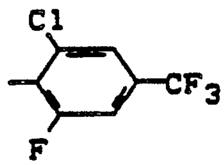 | Fp:95° C |
| 28 | $NO_2$ | H | -CO-H | 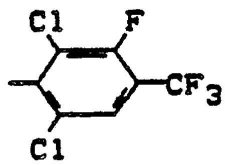 | Fp:159-171° C |
| 29 | $NO_2$ | H | $-CO-OCH_3$ | 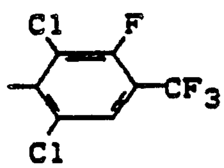 | Fp:91-104° C |
| 30 | $NO_2$ | H | $-CO-CH_2-OCH_3$ | 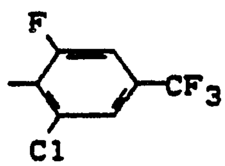 | Fp:144-146° C |
| 31 | $NO_2$ | H | $-CO-OCH_3$ | 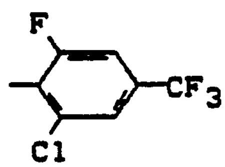 | $^1H$-NMR*) 8,32 |

**Tabelle 1** (Fortsetzung)

| Bsp. Nr. | $R^1$ | $R^2$ | $R^3$ | Ar | physikalische Eigenschaften |
|---|---|---|---|---|---|
| 32 | $NO_2$ | H | $-CO-CH_3$ | 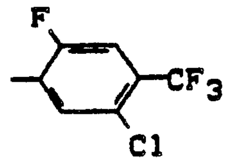 | Fp:154-157° C |
| 33 | $NO_2$ | H | -CO-H | 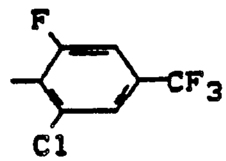 | Fp:60-65° C |
| 34 | $NO_2$ | H | $-CO-(CH_2)_2-CH_3$ | 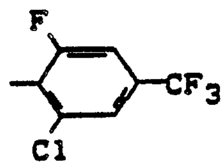 | Fp:68-73° C |
| 35 | H | H | $-CO-CH_3$ | 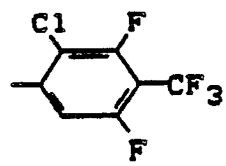 | Fp:49-55° C |
| 36 | $NO_2$ | H | $-CO-CH_3$ | 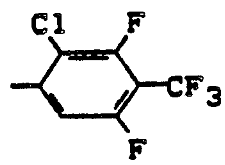 | Fp:85-95° C |
| 37 | H | H | $-CO-\overset{Cl}{\overset{\mid}{C}H}-CH_3$ | 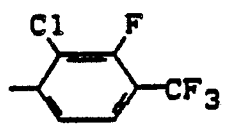 | Fp:97-100° C |
| 38 | H | H | $-CO-\overset{Cl}{\overset{\mid}{C}H}-CH_3$ | 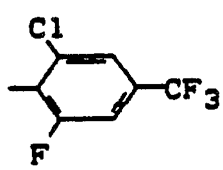 | Fp:107-108° C |

T a b e l l e 1 (Fortsetzung)

| Bsp. Nr. | $R^1$ | $R^2$ | $R^3$ | Ar | physikalische Eigenschaften |
|---|---|---|---|---|---|
| 39 | $NO_2$ | H | $-CO-\overset{Cl}{\overset{\vert}{C}}H-CH_3$ | 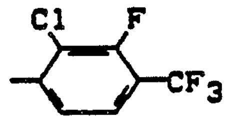 | Fp:83-86° C |
| 40 | $NO_2$ | H | $-CO-\overset{Cl}{\overset{\vert}{C}}H-CH_3$ | 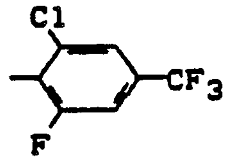 | $^1H$-NMR*) 8,38 |
| 41 | H | H | $-CO-CH_2-OCH_3$ | 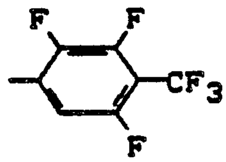 | Fp:58-61° C |
| 42 | $NO_2$ | H | $-CO-CH_2-OCH_3$ | 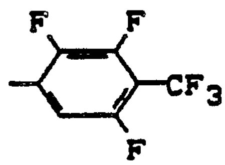 | Fp:97-98° C |
| 43 | $NO_2$ | H | $-CO-C_2H_5$ | 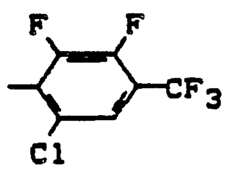 | Fp:126° C |
| 44 | $NO_2$ | H | $-CO-C_2H_5$ | 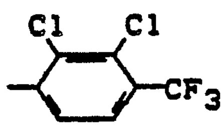 | Fp:91° C |
| 45 | $NO_2$ | H | $-CO-CH_2-OCH_3$ | 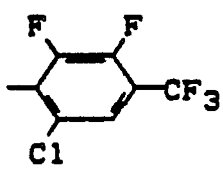 | Fp:99° C |
| 46 | $NO_2$ | H | $-CO-CH_2-OCH_3$ | 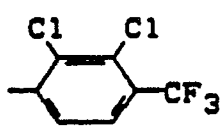 | $^1H$-NMR*) 3,5(s) |

**Tabelle 1** (Fortsetzung)

| Bsp. Nr. | $R^1$ | $R^2$ | $R^3$ | Ar | physikalische Eigenschaften |
|---|---|---|---|---|---|
| 47 | $NO_2$ | H | $-CO-CH_3$ | 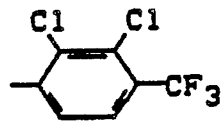 | Fp:109-117° C |
| 48 | H | H | $-CO-\overset{Cl}{\overset{\mid}{C}}H-CH_3$ | 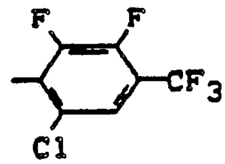 | $^1H$-NMR*) 4,45 (q) |
| 49 | H | H | $-CO-\overset{Cl}{\overset{\mid}{C}}H-CH_3$ | 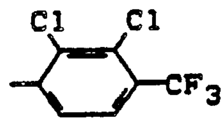 | Fp:122-127° C |
| 50 | $NO_2$ | H | $-CO-\overset{Cl}{\overset{\mid}{C}}H-CH_3$ | 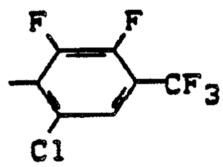 | Fp:105-197° C |
| 51 | $NO_2$ | H | $-CO-\overset{Cl}{\overset{\mid}{C}}H-CH_3$ | 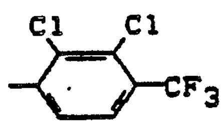 | Fp:120-122° C |
| 52 | H | H | $-CO-\overset{CH_3}{\overset{\mid}{C}}H-OC_2H_5$ | 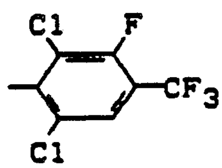 | $^1H$-NMR*) 6,7(d) |
| 53 | H | H | $-CO-\overset{CH_3}{\overset{\mid}{C}}H-OC_2H_5$ | 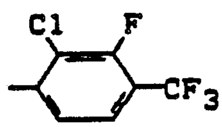 | Fp:68-71° C |
| 54 | H | H | $-CO-\overset{CH_3}{\overset{\mid}{C}}H-OC_2H_5$ | 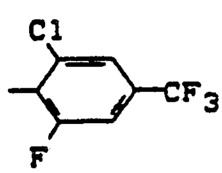 | $^1H$-NMR*) 6,7(t) |

**Tabelle 1** (Fortsetzung)

| Bsp. Nr. | $R^1$ | $R^2$ | $R^3$ | Ar | physikalische Eigenschaften |
|---|---|---|---|---|---|
| 55 | H | H | 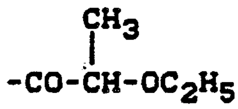 | 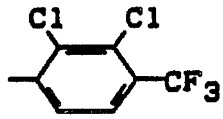 | Fp: 94-96° C |
| 56 | $NO_2$ | H | 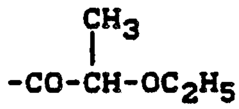 | 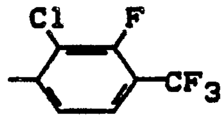 | Fp: 101-103° C |
| 57 | $NO_2$ | H | 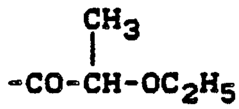 | 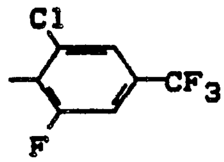 | Fp: 82-86° C |
| 58 | $NO_2$ | H | 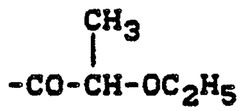 | 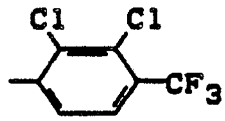 | Fp: 124-126° C |
| 59 | $NO_2$ | H | 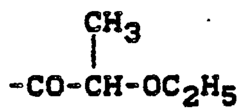 | 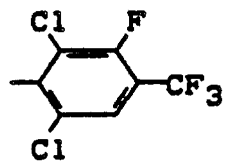 | Fp: 76-86° C |
| 60 | H | H | 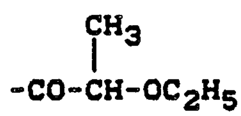 | 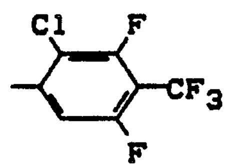 | Fp: 84-88° C |
| 61 | $NO_2$ | H | 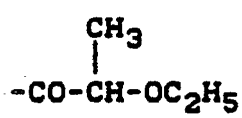 | 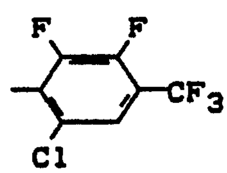 | Fp: 93-96° C |
| 62 | H | H | 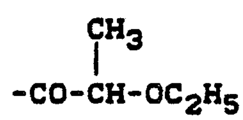 | 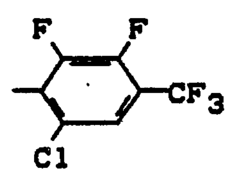 | $^1H$-NMR*) 7,78(d) |

T a b e l l e 1 (Fortsetzung)

| Bsp. Nr. | $R^1$ | $R^2$ | $R^3$ | Ar | physikalische Eigenschaften |
|---|---|---|---|---|---|
| 63 | $NO_2$ | H | $CH_3$<br>$-CO-CH-OC_2H_5$ | 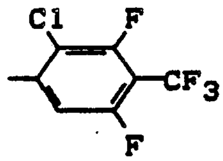 | $^1H$-NMR*) 8,33(s) |
| 64 | H | H | $-CO-CH_2OCH_2CF_3$ | 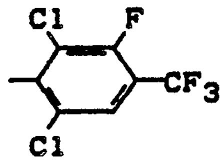 | Fp:96-102° C |
| 65 | $NO_2$ | H | $-CO-CH_2CH_2Cl$ | 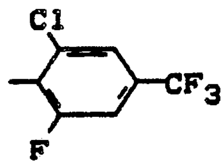 | Fp:93-96° C |
| 66 | $NO_2$ | H | $-CO-CH_2CH_2Cl$ | 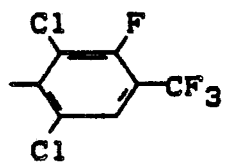 | Fp:118-124° C |
| 67 | $NO_2$ | H | $-CO-CH_2CH_2Cl$ | 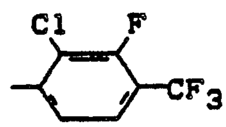 | Öl |
| 68 | $NO_2$ | H | $-CO-CH_2CH_2Cl$ | 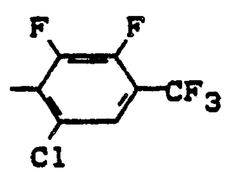 | Fp:138-141° C |
| 69 | $NO_2$ | H | $-CO-CH_2CH_2Cl$ | 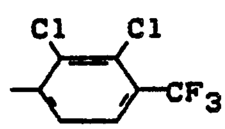 | Fp:106°-109° C |
| 70 | $NO_2$ | H | $-CO-CH_2CH_2Cl$ | 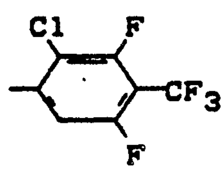 | Öl |

T a b e l l e 1 (Fortsetzung)

| Bsp. Nr. | $R^1$ | $R^2$ | $R^3$ | Ar | physikalische Eigenschaften |
|---|---|---|---|---|---|
| 71 | $NO_2$ | H | 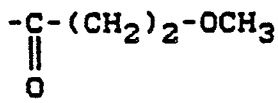 | 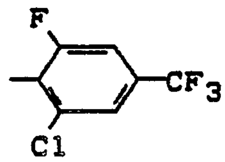 | Fp.103-106° C |
| 72 | $NO_2$ | H | 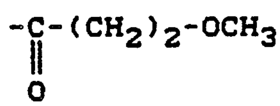 | 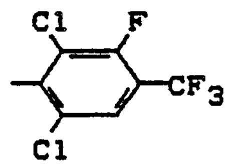 | Fp:80-85° C |
| 73 | $NO_2$ | H | 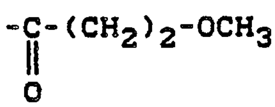 | 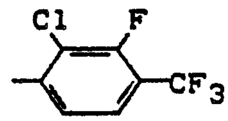 | Fp:122-126° C |
| 74 | $NO_2$ | H | 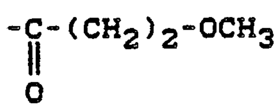 | 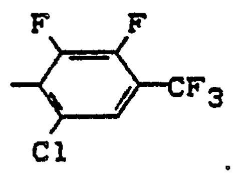 | $^1$H-NMR*): 8,34 |
| 75 | $NO_2$ | H | 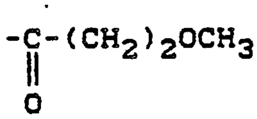 | 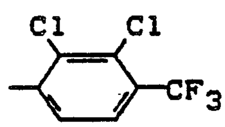 | Fp:125-128° C |
| 76 | $NO_2$ | H | 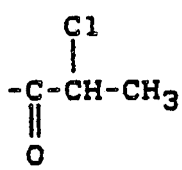 | 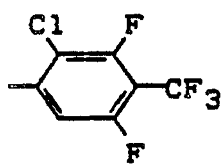 | Fp:93-98° C |
| 77 | $NO_2$ | H | 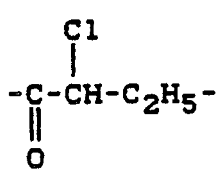 | 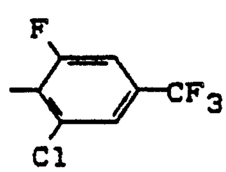 | $^1$H-NMR*): 8,37 |

T a b e l l e 1 (Fortsetzung)

| Bsp. Nr. | $R^1$ | $R^2$ | $R^3$ | Ar | physikalische Eigenschaften |
|---|---|---|---|---|---|
| 78 | $NO_2$ | H | 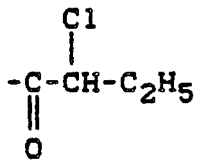 | 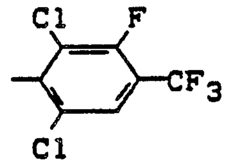 | Fp.78-87° C |
| 79 | $NO_2$ | H | 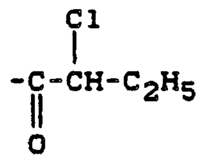 | 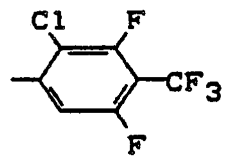 | Fp:100-104° C |
| 80 | $NO_2$ | H | 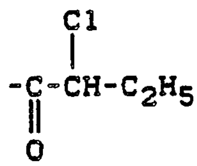 | 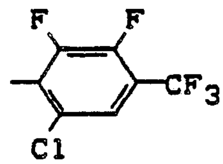 | Fp:93-99° C |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Herstellung der Ausgangsverbindungen:

Beispiel II-1:

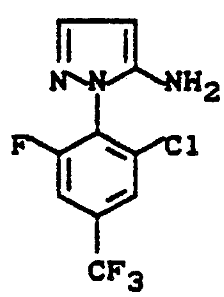

250 g (0,71 Mol) 5-Amino-4-ethoxycarbonyl-1-(2-chlor-6-fluor-4-trifluormethylphenyl)-pyrazol werden in 650 ml 50-%iger wässriger Schwefelsäure 2 Stunden auf 120° C erhitzt, wobei flüchtige Bestandteile abdestillieren. Man erhitzt für weitere 5 Stunden auf 115° C bis 120° C, kühlt dann ab, gibt 3 l Eiswasser zu,

stellt mit wäßriger Natronlauge auf pH 8 bis 9 ein, filtriert den entstandenen Niederschlag ab, wäscht mit Wasser nach und trocknet bei 50 °C im Vakuum.
Man erhält 163 g (82 % der Theorie) an 5-Amino-1-(2-chlor-6-fluor-4-trifluormethylphenyl)-pyrazol vom Schmelzpunkt 87-90 °C.

In entsprechender Weise erhält man die folgenden 1-Arylpyrazole der allgemeinen Formel (II):

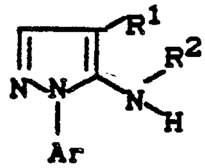

(II)

Tabelle 2

| Bsp. Nr. | $R^1$ | $R^2$ | Ar | Schmelzpunkt /° C |
|---|---|---|---|---|
| II-2 | H | H | 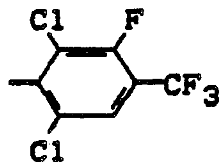 | 69-79 |
| II-3 | H | H | 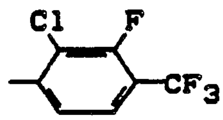 | 112-114 |
| II-4 | H | H | 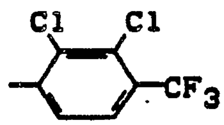 | 139-140 |
| II-5 | H | H | 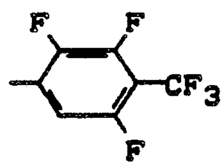 | 116-117 |
| II-6 | H | H | 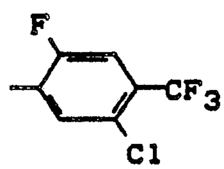 | 62-64 |
| II-7 | H | H | 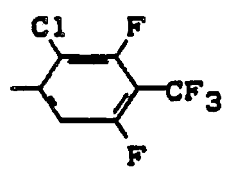 | 93-97 |
| II-8 | H | H | 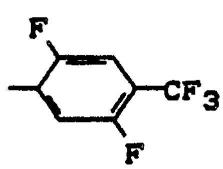 | 95-97 |
| II-9 | H | H | 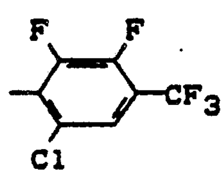 | 85-89 |

Beispiel XIV-1:

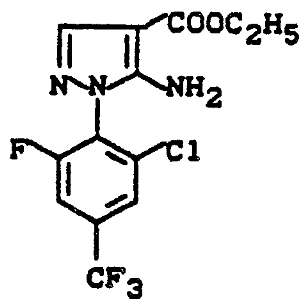

200 g (0,88 Mol) 2-Chlor-6-fluor-4-trifluormethylphenylhydrazin und 151 g (0,88 Mol) Ethoxymethylencyanessigsäureethylester in 400 ml Ethanol werden 30 Stunden auf Rückflußtemperatur erwärmt, dann auf ca. die Hälfte des Volumens eingeengt, abgekühlt, der so erhaltene Niederschlag abfiltriert, mit wenig kaltem Ethanol gewaschen und getrocknet.

Man erhält 250 g (81 % der Theorie) an 5-Amino-1-(2-chlor-6-fluor-4-trifluormethylphenyl)-pyrazol-4-yl-carbonsäureethylester vom Schmelzpunkt 147-149 °C.

In entsprechender Weise und gemäß den allgemeinen Angaben zur Herstellung erhält man die folgenden 1-Arylpyrazol-4-carbonsäureester der allgemeinen Formel (XIV):

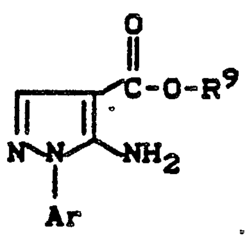

(XIV)

## Tabelle 3

| Bsp. Nr. | $R^9$ | Ar | physikalische Eigenschaften |
|---|---|---|---|
| XIV-2 | $C_2H_5$ | 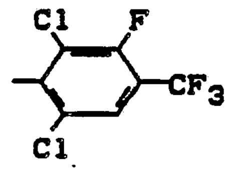 | Fp:70-74° C |
| XIV-3 | $C_2H_5$ | 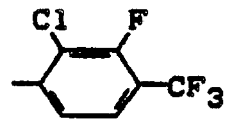 | Fp:119-126° C |
| XIV-4 | $C_2H_5$ | 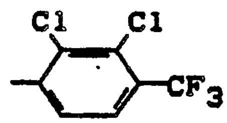 | Fp:174-175° C |
| XIV-5 | $C_2H_5$ | 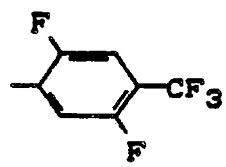 | Fp:132-133° C |
| XIV-6 | $C_2H_5$ | 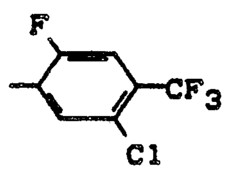 | Fp:139° C |
| XIV-7 | $C_2H_5$ | 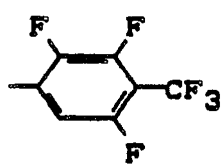 | $^1H$-NMR*) 7,87 |
| XIV-8 | $C_2H_5$ | 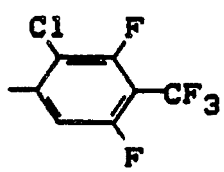 | Fp:171-175° C |
| XIV-9 | $C_2H_5$ | 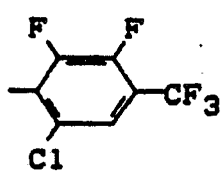 | Fp:88° C |

Beispiel XII-1:

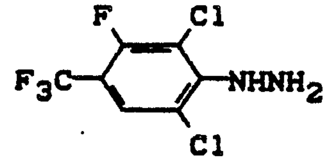

In 1000 ml Ethanol werden 470 g (1,87 Mol) 3,5-Dichlor-2,4-difluor-benzotrifluorid vorgelegt und 142 g (2,84 Mol) Hydrazinhydrat zudosiert und anschließend für 3 Stunden zum Rückfluß erhitzt. Danach wird das Lösungsmittel unter reduziertem Druck abdestilliert und der Rückstand in 1000 ml kaltes Wasser eingerührt. Nach 30 Minuten wird abgesaugt und das Festprodukt im Umluftschrank getrocknet.
Man erhält 445 g (90 % der Theorie) 2,6-Dichlor-3-fluor-4-trifluormethyl-phenylhydrazin mit einem Schmelzpunkt von 50 bis 51 °C.

Beispiel XII-2:

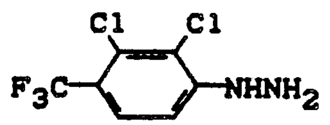

Es werden 100 g (0,4 Mol) 2,3,4-Trichlorbenzotrifluorid vorgelegt, 200 ml Pyridin und anschließend 100 g (2,0 Mol) Hydrazinhydrat zugefügt und dann 12 Stunden auf Rückflußtemperatur erhitzt. Danach wird das Pyridin zu 90 % abdestilliert und der verbleibende Rückstand in 250 ml Wasser eingerührt. Das kristalline Produkt wird abge saugt, mit etwas Wasser gewaschen und getrocknet. Man erhält 78 g (80 % der Theorie) 2,3-Dichlor-4-trifluormethyl-phenylhydrazin mit einem Schmelzpunkt von 79 bis 80 °C.

In entsprechender Weise und gemäß den allgemeinen Angaben zur Herstellung erhält man die folgenden Arylhydrazine der Formel (XII),

$$Ar - NH - NH_2 \qquad (XII)$$

T a b e l l e 4

| Bsp. Nr. | Ar | Schmelzpunkt /°C |
|---|---|---|
| XII-3 | 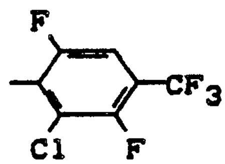 | 72-83 |
| XII-4 | 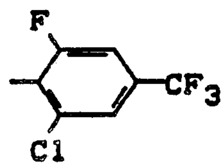 | 60-61 |
| XII-5 | 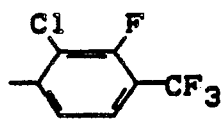 | 102-103 |
| XII-6 | 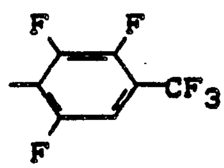 | 60-62 |
| XII-7 | 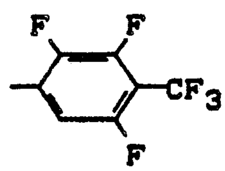 | 69-70 |

**T a b e l l e 4** (Fortsetzung)

| Bsp. Nr. | Ar | Schmelzpunkt /° C |
|---|---|---|
| XII-8 | 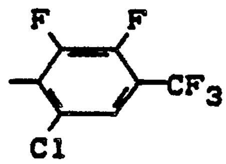 | 80 |
| XII-9 | 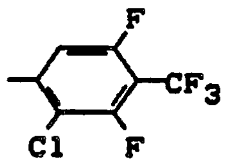 | 82-84 |
| XII-10 | 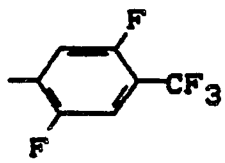 | 41 |
| XII-11 | 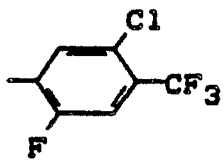 | 69-70 |

Beispiel XI-1/XI-2:

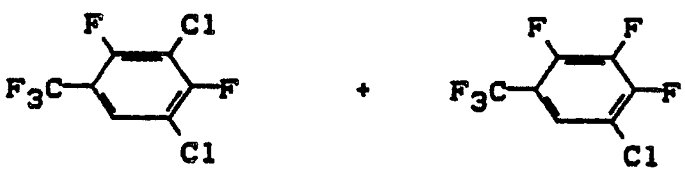

Zu 800 g (13,8 Mol) Kaliumfluorid in 1800 ml trockenem Tetramethylensulfon gibt man 900 g (3,17 Mol) 2,3,4,5-Tetrachlorbenzotrifluorid (vgl. z.B. EP 150 587) und erhitzt 10 Stunden auf 200 °C. Zur Aufarbeitung wird im Vakuum eingeengt und destilliert.

Man erhält zunächst 50 g (6,8 % der Theorie) an 2,3,4-Trifluor-5-chlor-benzotrifluorid vom Siedepunkt Kp 31-38 °C bei 10 mbar und vom Brechungsindex $n_D^{20}$ = 1,4130 und als 2.Fraktion 490 g (61,6 % der Theorie) an 2,4-Difluor-3,5-dichlorbenzotrifluorid vom Siedepunkt 57-59 °C bei 10 mbar und vom Brechungsindex $n_D^{20}$ = 1,4510.

Beispiel XI-3:

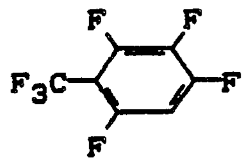

252,5 g (1 Mol) 3-Chlor-2,4,5,6-tetrafluorbenzotrifluorid (vergl. z.B. Zh. obshch. Khim. 37, 1686-1687 [1967]) und 86 g (1,05 Mol) Natriumacetat in 1000 ml Eisessig werden in Gegenwart von 10 g Palladium auf Aktivkohle (5%ig) bei einem Wasserstoffdruck von 30 bis 50 bar und 120 °C bis zur Druckkonstanz hydriert. Zur Aufarbeitung filtriert man aus der erkalteten Reaktionsmischung den Katalysator ab und destilliert den Rückstand.

Man erhält 210 g (96 % der Theorie) an 2,3,4,6-Tetrafluorbenzotrifluorid vom Siedepunkt 105-106 °C und vom Brechungsindex $n_D^{20}$ = 1,3770.

In entsprechender Weise erhält man die folgenden Arylhalogenide der Formel (XI):

$$Ar - Hal^2 \qquad (XI)$$

**Tabelle 5**

| Bsp. Nr. | Ar | $Hal^2$ | Siedepunkt/mbar Brechungsindex $n_D^{20}$ |
|---|---|---|---|
| XI-4 | 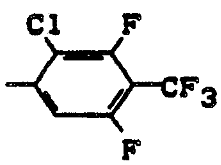 | F | Kp:145° C/760<br>$n_D^{20}$ = 1,4170 |
| XI-5 | 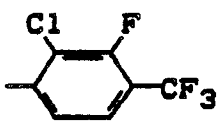 | F | Kp:37° C/16<br>$n_D^{20}$ = 1,4268 |
| XI-6 | 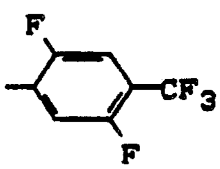 | F | Kp:104° C/760<br>$n_D^{20}$ = 1,3862 |
| XI-7 | 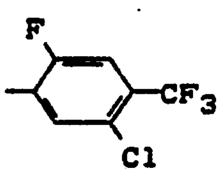 | F | Kp:130-140° C/760<br>$n_D^{20}$ = 1,4250 |

Anwendungsbeispiele:

In den folgenden Anwendungsbeispielen wurde die nachstehend aufgeführte Verbindung als Vergleichsubstanz eingesetzt:

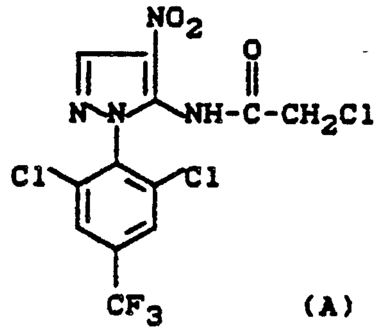

(A)

4-Nitro-5-chloracetamido-1-(2,6-dichlor-4-trifluormethylphenyl)-pyrazol
(bekannt aus DE-OS 34 02 308 / Beispiel Nr. 20).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber der Vergleichssubstanz (A) zeigen in diesem Test z.B. Die Verbindungen gemäß folgender Herstellungsbeispiele: 3, 4, 6, 8, 11, 13, 24, 26, 27, 29, 30, 31, 33, 34, 43, 45 und 46.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

Eine deutliche Überlegenheit in der Wirksamkeit gegenüber der Vergleichssubstanz (A) zeigen in diesem Test z.B. die Verbindungen gemäß folgender Herstellungsbeispiele: 3, 6, 13, 24, 25, 26, 28, 29, 30, 31, 32, 33, 34, 43, 44, 45, 46 und 47.

Beispiel C

Entlaubung und Austrocknung der Blätter bei Baumwolle

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall

\+ leichtes Austrocknen der Blätter, geringer Blattfall

\+ + starkes Austrocknen der Blätter, starker Blattfall

\+ + + sehr starkes Austrocknen der Blätter, sehr starker Blattfall

Eine deutliche Wirksamkeit im Vergleich zur unbehandelten Kontrolle zeigen in diesem Test beispielsweise die Verbindungen gemäß den Herstellungsbeispielen 1, 3, 6, 7, 8, 10, 11, 12, 19, 21, 24, 25, 26, 27, 28, 29, 30, 31, 32, 33, 34, 43, 44, 45 und 46.

## Ansprüche

1. 1-Arylpyrazole der Formel (I),

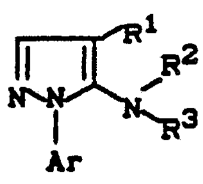

(I)

in welcher

$R^1$ für Wasserstoff oder Nitro steht,

$R^2$ für Wasserstoff, für einen Rest $-\overset{X}{\overset{||}{C}}-R^4$ oder einen Rest $-S(O)_n-R^5$ steht,

$R^3$ für Alkyl, für einen Rest $-\overset{X}{\overset{||}{C}}-R^4$ oder für einen Rest $-S(O)_n-R^5$ steht und außerdem für den Fall, daß $R^2$ für einen Rest $-SO_2-R^5$ steht, auch für ein salzartig gebundenes anorganisches oder organisches Kation steht,

wobei

$R^4$ für Wasserstoff, Alkyl, Alkenyl, Alkinyl, Monohalogenalkyl, Alkoxyalkyl, Halogen alkoxyalkyl, Alkylthioalkyl, Alkoxy, Alkylthio, Alkylsulfonylalkyl, Alkylsulfinylalkyl, Alkylamino, Dialkylamino, gegebenenfalls substituiertes Cycloalkyl, gegebenenfalls substituiertes Aryl, gegebenenfalls substituiertes Aryloxy, gegebenenfalls substituiertes Arylthio, oder für gegebenenfalls substituiertes Arylamino steht,
X für Sauerstoff oder Schwefel steht,
$R^5$ für Alkyl, Halogenalkyl oder für gegebenenfalls substituiertes Aryl steht,
n für eine Zahl 0,1 oder 2 steht und
Ar für einen der Reste

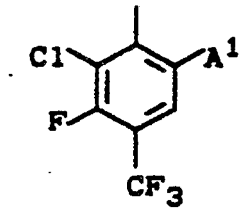 ; 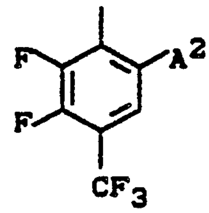 ; 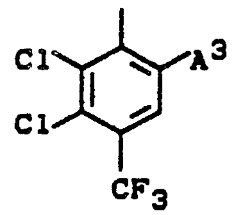 ;

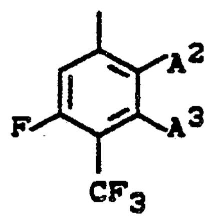 ; 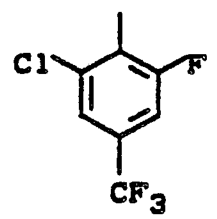 oder 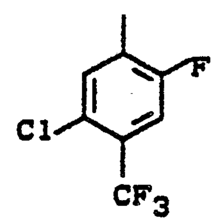

steht, wobei
$A^1$ für Wasserstoff, Fluor oder Chlor steht,
$A^2$ für Fluor oder Chlor steht und
$A^3$ für Wasserstoff oder Fluor steht,
wobei jedoch die Verbindungen 5-Ethoxycarbonylamino-1-(2,3-dichlor-4-trifluormethylphenyl)-4-nitro-pyrazol und 5-Acetamido-1-(2,5-difluor-4-trifluormethylphenyl)-pyrazol ausgenommen sind.

2. 1-Arylpyrazole der Formel (I) gemäß Anspruch 1,
in welcher
$R^1$ für Wasserstoff oder Nitro steht,
$R^2$ für Wasserstoff, für einen Rest $-\overset{X}{\overset{\|}{C}}-R^4$ oder für einen Rest $-S(O)_n-R^5$ steht,
$R^3$ für geradkettiges oder verzweigtes Alkyl mit 1 bis 6 Kohlenstoffatomen, für einen Rest $-\overset{X}{\overset{\|}{C}}-R^4$ oder für einen Rest $-S(O)_n-R^5$ steht und außerdem für den Fall, daß $R^2$ für einen Rest $-SO_2-R^5$ steht, auch für ein salzartig gebundenes Äquivalent eines Alkali- oder Erdalkali- oder Übergangsmetallkations oder für ein gegebenenfalls durch $C_1$-$C_6$-Alkyl oder Phenyl substituiertes Ammoniumion steht, wobei
$R^4$ für Wasserstoff, für geradkettiges oder verzweigtes Alkyl mit 1 bis 12 Kohlenstoffatomen, für jeweils geradkettiges oder verzweigtes Alkenyl oder Alkinyl mit jeweils 2 bis 6 Kohlenstoffatomen, für jeweils geradkettiges oder verzweigtes Monohalogenalkyl, Alkoxyalkyl, Halogenalkoxyalkyl, Alkylthioalkyl, Alkoxy, Alkylthio, Alkylsulfonylalkyl, Alkylsulfinylalkyl, Alkylamino oder Dialkylamino mit jeweils 1 bis 4 Kohlenstoffatomen in den einzelnen Alkylteilen und im Fall des Halogenalkoxyalkyl mit jeweils 1 bis 9 gleichen oder verschiedenen Halogenatomen steht, außerdem für gegebenenfalls einfach oder mehrfach, gleich oder verschieden durch Halogen und/oder jeweils geradkettiges oder verzweigtes Alkyl oder Halogenalkyl mit jeweils 1 bis 4 Kohlenstoffatomen und im Fall des Halogenalkyl mit 1 bis 9 gleichen oder verschiedenen Halogenatomen substituiertes Cycloalkyl mit 3 bis 7 Kohlenstoffatomen, sowie für jeweils gegebenenfalls einfach oder mehrfach, gleich oder

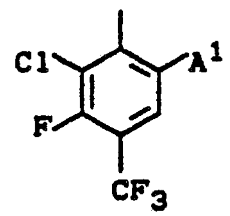 ; 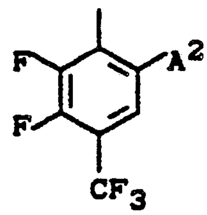 ; 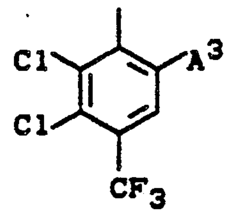 ;

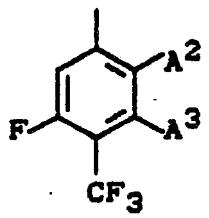 ; 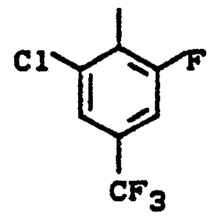 oder 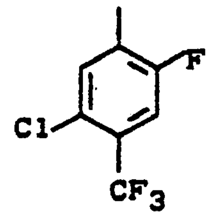

steht, wobei
$A^1$ für Wasserstoff, Fluor oder Chlor steht,
$A^2$ für Fluor oder Chlor steht und
$A^3$ für Wasserstoff oder Fluor steht,
wobei jedoch die Verbindungen 5-Ethoxycarbonylamino-1-(2,3-dichlor-4-trifluormethylphenyl)-5-nitro-pyrazol und 5-Acetamido-1-(2,5-difluor-4-trifluormethylphenyl)-pyrazol ausgenommen sind,

3. 1-Arylpyrazole der Formel (I) gemäß Anspruch 1,
in welcher
$R^1$ für Wasserstoff oder Nitro steht,
$R^2$ für Wasserstoff, für einen Rest $-\underset{\underset{X}{\|}}{C}-R^4$ oder für einen Rest $-S(O)_n-R^5$ steht,
$R^3$ für Methyl, Ethyl, n- oder i-Propyl, n-, i-, s- oder t-Butyl, für einen Rest $-\underset{\underset{X}{\|}}{C}-R^4$ oder für einen Rest $-S(O)_n-R^5$ steht, und außerdem für den Fall, daß $R^2$ für einen Rest $-SO_2-R^5$ steht, auch für ein salzartig gebundenes Äquivalent eines Natrium-, Kalium-, Magnesium-, Calcium-, Barium-, Kupfer-, Zink-, Mangan-, Zinn-, Eisen-, Cobalt- oder Nickel-ions steht, oder für ein gegebenen falls ein-bis dreifach, gleich oder verschieden durch Methyl, Ethyl, n- oder i-Propyl, n-, i-, s-oder t-Butyl oder Phenyl substituiertes Ammoniumion steht, wobei
$R^4$ für Wasserstoff, Methyl, Ethyl, n- oder i-Propyl, n-, i-, s- oder t-Butyl, für jeweils geradkettiges oder verzweigtes Pentyl, Hexyl, Heptyl, Octyl, Nonyl, Decyl, Undecyl, für Vinyl, Allyl, Propargyl, n- oder i-Butenyl, Methoxymethyl, Ethoxymethyl, Methoxyethyl, Ethoxyethyl, n-Propoxymethyl, Isopropoxymethyl, Methylthiomethyl, Methoxy, Ethoxy, Methylthio, Ethylthio, Methylsulfonylmethyl, Methylsulfonylethyl, Ethylsulfonylmethyl, Ethylsulfonylethyl, Methylsulfinylmethyl, Methylsulfinylethyl, Ethylsulfinylmethyl, Ethylsulfinylethyl, Methylamino, Ethylamino, Dimethylamino, Diethylamino, Chlormethyl, Iodmethyl, Brommethyl, 1-Chlorethyl, 2-Chlorethyl, 2-Bromethyl, 3-Chlorpropyl, für Trifluorethoxymethyl, für jeweils gegebenenfalls ein- bis vierfach, gleich oder verschieden durch Fluor, Chlor, Brom, Methyl oder Trifluormethyl substituiertes Cyclopropyl, Cyclopentyl oder Cyclohexyl oder für jeweils gegebenenfalls ein- bis dreifach, gleich oder verschieden durch Methyl, Methoxy, Chlor oder Trifluormethyl substituiertes Phenyl, Phenoxy, Phenylthio oder Phenylamino steht,
X für Sauerstoff oder Schwefel steht,
$R^5$ für Methyl, Ethyl, n- oder i-Propyl, n-, i-, s- oder t-Butyl, Chlormethyl, Dichlormethyl, Trichlormethyl, Trifluormethyl oder für gegebenenfalls ein- bis dreifach, gleich oder verschieden durch Methyl, Methoxy, Chlor oder Trifluormethyl substituiertes Phenyl steht,
n für eine Zahl 0, 1 oder 2 steht und
Ar für einen der Reste

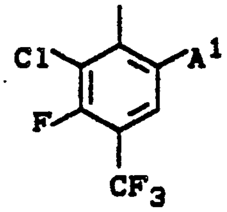 ; 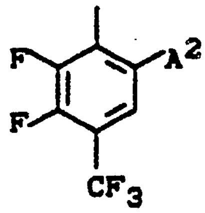 ; 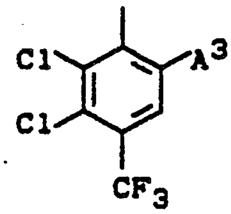 ;

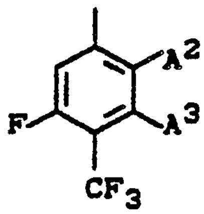 ; 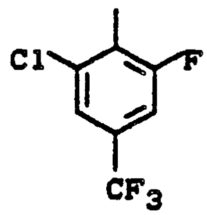 oder 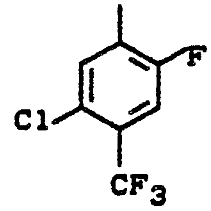

steht, wobei
$A^1$ für Wasserstoff, Fluor oder Chlor steht,
$A^2$ für Fluor oder Chlor steht und
$A^3$ für Wasserstoff oder Fluor steht,
wobei jedoch die Verbindungen 5-Ethoxycarbonylamino-1-(2,3-dichlor-4-trifluormethylphenyl)-4-nitro-pyrazol und 5-Acetamido-1-(2,5-difluor-4-trifluormethylphenyl)-pyrazol ausgenommen sind.

4. 1-Arylpyrazole der Formel (I) gemäß Anspruch 1,
in welcher
$R^1$ für Nitro steht,
$R^2$ für Wasserstoff steht,
$R^3$ für Methyl, Ethyl, n- oder i-Propyl, n-, i-, s- oder t-Butyl, für einen Rest $-\underset{\underset{O}{\|}}{C}-R^4$
oder für einen Rest $-SO_2-R^5$ steht, wobei
$R^4$ für Wasserstoff, Methyl, Ethyl, n- oder i-Propyl, n-Butyl, Allyl, Propargyl, n- oder i-Butenyl, für Methoxymethyl, Ethoxymethyl, n-Propoxymethyl, Isopropoxymethyl, Methoxyethyl, Ethoxyethyl, Methylthiomethyl, Methoxy, Ethoxy, Methylthio, Ethylthio, Methylamino, Ethylamino, Dimethylamino, Diethylamino, Chlormethyl, Iodmethyl, Brommethyl, 1-Chlorethyl, 2-Chlorethyl, 2-Bromethyl, 1-Chlorpropyl, 3-Chlorpropyl, für Trifluorethoxymethyl, für Cyclopropyl, Cyclopentyl oder Cyclohexyl oder für jeweils gegebenenfalls ein- bis dreifach, gleich oder verschieden durch Methyl, Methoxy, Chlor oder Trifluormethyl substituiertes Phenyl, Phenoxy, Phenylthio oder Phenylamino steht,
$R^5$ für Methyl, Ethyl, n- oder i-Propyl, n-, i-, s- oder t-Butyl, Chlormethyl, Dichlormethyl, Trichlormethyl, Trifluormethyl oder für gegebenenfalls ein- bis dreifach, gleich oder verschieden durch Methyl, Methoxy, Chlor oder Trifluormethyl substituiertes Phenyl steht und
Ar für einen der Reste

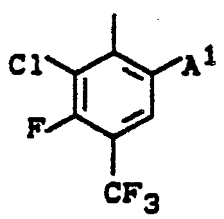 ; 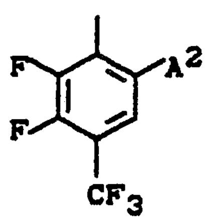 ; 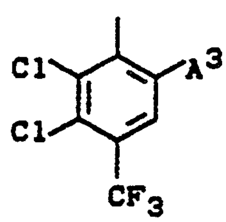 ;

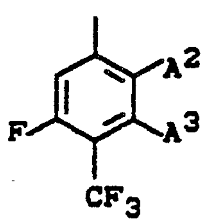 ; 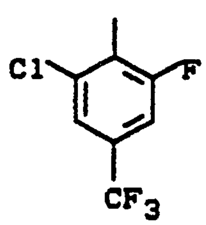 oder 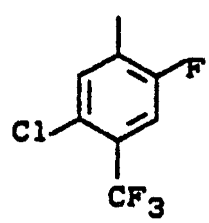

steht, wobei
$A^1$ für Wasserstoff, Fluor oder Chlor steht,
$A^2$ für Fluor oder Chlor steht und
$A^3$ für Wasserstoff oder Fluor steht,
wobei jedoch die Verbindungen 5-Ethoxycarbonylamino-1-(2,3-dichlor-4-trifluormethylphenyl)-4-nitro-pyrazol und 5-Acetamido-1-(2,5-difluor-4-trifluormethylphenyl)-pyrazol ausgenommen sind.

5. Verfahren zur Herstellung von 1-Arylpyrazolen der allgemeinen Formel (I),

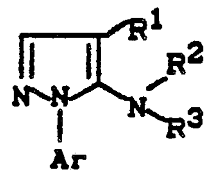

(I)

in welcher
$R^1$ für Wasserstoff oder Nitro steht,
$R^2$ für Wasserstoff, für einen Rest $-\overset{X}{\overset{\|}{C}}-R^4$ oder einen Rest $-S(O)_n-R^5$ steht,
$R^3$ für Alkyl, für einen Rest $-\overset{X}{\overset{\|}{C}}-R^4$ oder für einen Rest $-S(O)_n-R^5$ steht und außerdem für den Fall, daß $R^2$ für einen Rest $-SO_2-R^5$ steht, auch für ein salzartig gebundenes anorganisches oder organisches Kation steht,
wobei
$R^4$ für Wasserstoff, Alkyl, Alkenyl, Alkinyl, Monohalogenalkyl, Alkoxyalkyl, Halogenalkoxyalkyl, Alkylthioalkyl, Alkyl sulfonylalkyl, Alkylsulfinylalkyl, Alkoxy, Alkylthio für gegebenenfalls substituiertes Cycloalkyl, für gegebenenfalls substituiertes Aryl, für gegebenenfalls substituiertes Aryloxy, für gegebenenfalls substituiertes Arylthio, für Alkylamino, Dialkylamino oder für gegebenenfalls substituiertes Arylamino steht,
X für Sauerstoff oder Schwefel steht,
$R^5$ für Alkyl, Halogenalkyl oder für gegebenenfalls substituiertes Aryl steht,
n für eine Zahl 0, 1 oder 2 steht und
Ar für einen der Reste

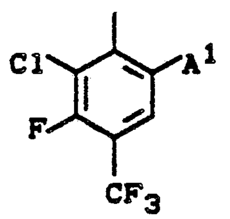 ; 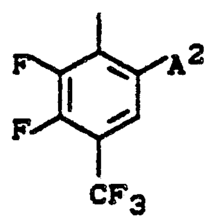 ; 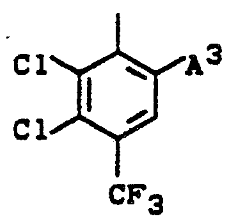 ;

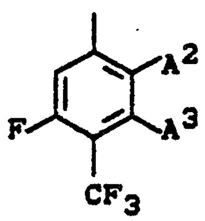 ; 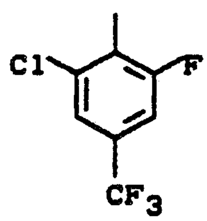 oder 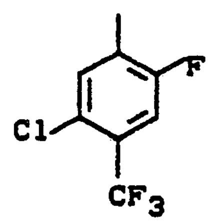

steht, wobei
$A^1$ für Wasserstoff, Fluor oder Chlor steht,
$A^2$ für Fluor oder Chlor steht und
$A^3$ für Wasserstoff oder Fluor steht,
wobei jedoch die Verbindungen 5-Ethoxycarbonylamino-1-(2,3-dichlor-4-trifluormethylphenyl)-4-nitro-pyrazol und 5-Acetamido-1-(2,5-difluor-4-trifluormethylphenyl)-pyrazol ausgenommen sind,
dadurch gekennzeichnet, daß man

(a) 1-Arylpyrazoleder Formel (Ia),

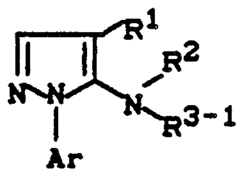

(Ia)

in welcher

$R^{3-1}$ für Alkyl, für einen Rest $-\overset{X}{\overset{\|}{C}}-R^4$ oder für einen Rest $-S(O)_n-R^5$ steht und $R^1$, $R^2$, $R^4$, $R^5$, X, n und Ar die oben angegebene Bedeutung haben, erhält, wenn man 1-Arylpyrazole der Formel (II),

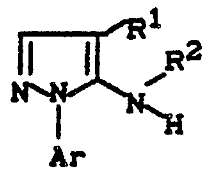

(II)

in welcher

$R^1$, $R^2$ und Ar die oben angegebene Bedeutung haben,

(a-α) mit Acylierungsmitteln der Formel (III),

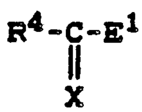

(III)

in welcher

$E^1$ für eine elektronenanziehende Abgangsgruppe steht und

$R^4$ und X die oben angegebene Bedeutung haben, oder

(a-β) mit Sulfenylierungs-, Sulfinylierungs- oder Sulfonylierungsmitteln der Formel (IV),

$R^5-S(O)_n-E^2$ (IV)

in welcher

$E^2$ für eine elektronenanziehende Abgangsgruppe steht und

$R^5$ und n die oben angegebene Bedeutung haben, oder

(a-γ) mit Alkylierungsmitteln der Formel (V),

$R^{3-2}-E^3$ (V)

in welcher

$R^{3-2}$ für Alkyl steht und

$E^3$ für eine elektronenanziehende Abgangsgruppe steht,

jeweils gegebenenfalls in Gegenwart eines Verdünnungsmittels sowie gegebenenfalls in Gegenwart eines Reaktionshilfsmittels umsetzt; oder daß man

(b) 1-Arylpyrazole der Formel (Ib),

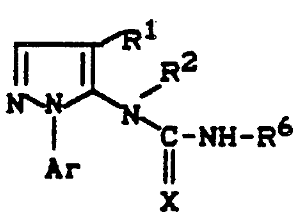

(Ib)

in welcher
$R^6$ für Alkyl oder für gegebenenfalls substituiertes Aryl steht und
$R^1$, $R^2$, X und Ar die oben angegebene Bedeutung haben,
erhält, wenn man 1-Arylpyrazole der Formel (II),

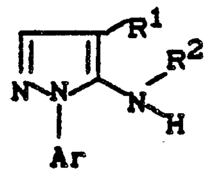

(II)

in welcher
$R^1$, $R^2$ und Ar die oben angegebene Bedeutung haben,
mit Iso(thio)cyanaten der Formel (VI),

$R^6$-N=C=X (VI)

in welcher
$R^6$ und X die oben angegebene Bedeutung haben,
gegebenenfalls in Gegenwart eines Verdünnungsmittels und gegebenenfalls in Gegenwart eines Reaktionshilfsmittels umsetzt; oder daß man

(c) 1-Arylpyrazole der Formel (Ic),

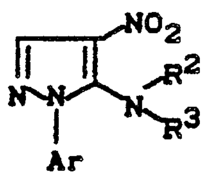

(Ic)

in welcher
$R^2$, $R^3$ und Ar die oben angegebene Bedeutung haben,
erhält, wenn man 1-Arylpyrazole der Formel (Iz),

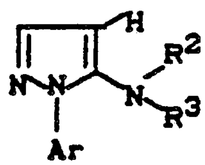

(Iz)

in welcher
$R^2$, $R^3$ und Ar die oben angegebene Bedeutung haben,
mit Salpetersäure gegebenenfalls in Gegenwart eines Verdünnungsmittels und gegebenenfalls in Gegenwart eines Reaktionshilfsmittels umsetzt; oder daß man

(d) 1-Arylpyrazole der Formel (Id),

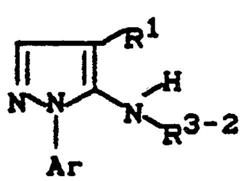

(Id)

in welcher
$R^{3-2}$ für Alkyl steht und
$R^1$ und Ar die oben angegebene Bedeutung haben,

erhält, wenn man 1-Arylpyrazole der Formel (Iy),

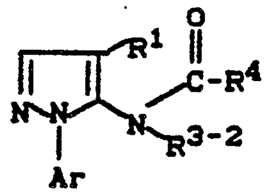

(Iy)

in welcher
$R^1$, $R^{3-2}$, $R^4$ und Ar die oben angegebene Bedeutung haben,
gegebenenfalls in Gegenwart eines Verdünnungsmittels und gegebenenfalls in Gegenwart eines Reaktionshilfsmittels an der Aminogruppe in 5-Position des Pyrazolringes hydrolysiert; oder daß man

(e) 1-Arylpyrazole der Formel (Ie),

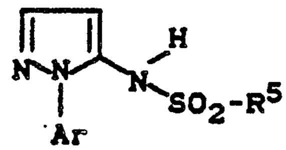

(Ie)

in welcher
$R^5$ und Ar die oben angegebene Bedeutung haben,
erhält, wenn man 1-Arylpyrazole der Formel (Ix),

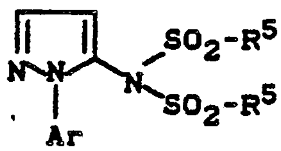

(Ix)

in welcher
$R^5$ und Ar die oben angegebene Bedeutung haben,
gegebenenfalls in Gegenwart eines Verdünnungsmittels und gegebenenfalls in Gegenwart eines Reaktionshilfsmittels an der Aminogruppe in 5-Position des Pyrazolringes hydrolysiert; oder daß man

(f) 1-Arylpyrazole der Formel (If),

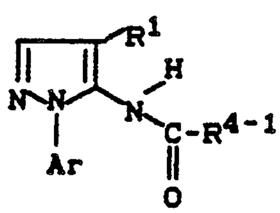

(If)

in welcher
$R^{4-1}$ für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht und
$R^1$ und Ar die oben angegebene Bedeutung haben,
erhält, wenn man 1-Arylpyrazole der Formel (Iw),

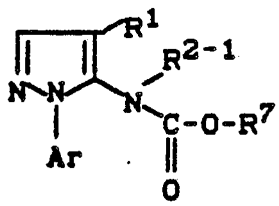

(Iw)

in welcher
$R^{2-1}$ für Wasserstoff oder für einen Rest $-\underset{\underset{O}{\|}}{C}-O-R^7$ steht,
$R^7$ für gegebenenfalls substituiertes Aryl steht und
$R^1$ und Ar die oben angegebene Bedeutung haben,
mit Alkoholen, Aminen oder Thiolen der Formel(VII),

$R^{4-1}$-H (VII)

in welcher
$R^{4-1}$ die oben angegebene Bedeutung hat,
gegebenenfalls in Gegenwart eines Verdünnungsmittels und gegebenenfalls in Gegenwart eines Reaktionshilfsmittels umsetzt; oder daß man

(g) 1-Arylpyrazole der Formel (Ig),

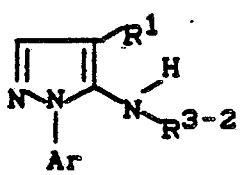

(Ig)

in welcher
$R^{3-2}$ für Alkyl steht und
$R^1$ und Ar die oben angegebene Bedeutung haben,
erhält, wenn man 5-Halogen-1-aryl-pyrazole der Formel (VIII),

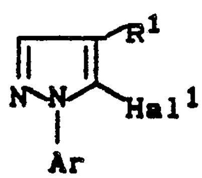

(VIII)

in welcher
$Hal^1$ für Halogen steht und
$R^1$ und Ar die oben angegebene Bedeutung haben,
mit Aminen der Formel (IX),

$H_2N$ - $R^{3-2}$ (IX)

in welcher
$R^{3-2}$ die oben angegebene Bedeutung hat,
gegebenenfalls in Gegenwart eines Verdünnungsmittels und gegebenenfalls in Gegenwart eines Reaktionshilfsmittels umsetzt; oder daß man

(h) 1-Arylpyrazole der Formel (Ih),

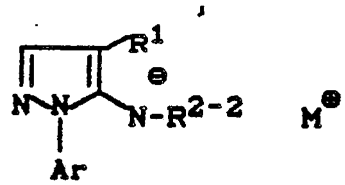

(Ih)

in welcher
$R^{2-2}$ für einen Rest $-SO_2-R^5$ steht,
$M^{\oplus}$ für ein Äquivalent eines anorganischen oder organischen Kations steht und
$R^1$, $R^5$ und Ar die oben angegebene Bedeutung haben,
erhält, wenn man 1-Arylpyrazole der Formel (Iv),

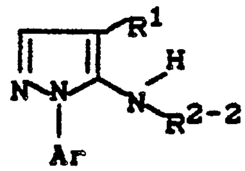

(Iv)

in welcher
$R^1$, $R^{2-2}$ und Ar die oben angegebene Bedeutung haben,
mit Salzen der Formel (X),

$M^{\oplus}$-$G^{\ominus}$ (X)

in welcher
$M^{\oplus}$ die oben angegebene Bedeutung hat und
$G^{\ominus}$ für ein Äquivalent eines geeigneten Gegenions steht,
oder mit primären, sekundären oder tertiären Aminen, gegebenenfalls in Gegenwart eines Verdünnungsmittels umsetzt.

6. Herbizide und pflanzenwuchsregulierende Mittel, gekennzeichnet, durch einen Gehalt an mindestens einem 1-Arylpyrazol der Formel (I) gemäß den Ansprüchen 1 bis 5.

7. Verfahren zur Bekämpfung von Unkräutern, dadurch gekennzeichnet, daß man 1-Arylpyrazole der Formel (I) gemäß den Ansprüchen 1 bis 5 auf die Unkräuter und/oder ihren Lebensraum einwirken läßt.

8. Verwendung von 1-Arylpyrazolen der Formel (I) gemäß Ansprüchen 1 bis 5 zur Bekämpfung von Unkräutern und/oder als Pflanzenwachstumsregulatoren.

9. Verfahren zur Herstellung von herbiziden und/oder pflanzenwuchsregulierenden Mitteln, dadurch gekennzeichnet, daß man 1-Arylpyrazole der Formel (I) gemäß den Ansprüchen 1 bis 5 mit Streckmitteln und/oder oberflächenaktiven Substanzen vermischt.